# EUROPEAN PATENT APPLICATION

(11) **EP 1 775 293 A1**
(43) Date of publication of application: **18.04.2007**
(21) Application number: 05768939.0
(22) Date of filing: 05.08.2005
(51) Int. Cl.: C07D 401/14, A61K 31/501, A61P 7/02, A61P 9/10

(54) **ANTIPLATELET AGENT AND PROCESS FOR PRODUCING THE SAME**

(30) Priority: 06.08.2004 JP 2004230011
(71) Applicant: DAIICHI PHARMACEUTICAL CO., LTD., Chuo-ku, Tokyo 103-8234 (JP)
(72) Inventor: SATO, Koji c/o Daiichi Pharmaceutical Co., Ltd., Edogawa-ku, Tokyo 1348630 (JP); YOSHIDA, Syouko Daiichi Pharmaceutical Co., Ltd., Edogawa-ku, Tokyo 1348630 (JP); YAGI, Tsutomu c/o Daiichi Pharmaceutical Co., Ltd., Edogawa-ku, Tokyo 1348630 (JP); SAKURATANI, Kenji Daiichi Pharmaceutical Co., Ltd., Edogawa-ku, Tokyo 1348630 (JP)
(74) Representative: Hartz, Nikolai
(86) International application number: PCT/JP2005/014405
(87) International publication number: WO 2006/013962

(57) **Abstract**

A compound having an action of suppressing platelet aggregation isprovided. This compound also exhibits excellent physical properties and oral absorbability. This compound is represented by formula (Ib): Its production method is also provided.

## Description

### Technical Field

This invention relates to a pyrazole derivative which is useful as a platelet aggregation inhibitor, and its production method.

### Background Art

Platelets play an important role of aggregating and forming thrombus to stop the bleeding when a blood vessel is damaged. On the other hand, platelets aggregate to form thrombus and embolus in the part of the blood vessel where vascular endothelium is damaged or constricted. The thrombus and embolus formed will cause ischemic diseases such as myocardial infarction, angina pectoris, ischemic cerebro vascular disorder, and peripheral vascular disorder. In view of such situation, platelet aggregation inhibitors have been used for preventing and treating ischemic diseases. Among such platelet aggregation inhibitors, low dose aspirin has been used for a long time, and its effect has been proved by APT (Antiplatelet Trialists' Collaboration) which has conducted metaanalysis of a plurality of clinical trials in which aspirin was administered to 100, 000 patients (see Non-Patent Document 1).
Aspirin, however, is known to induce the so called "aspirin ulcer", a side effect inducing gastrointestinal bleeding. This side effect occurs at a rate of 1 per 100 patients independent of the amount administered (see Non-Patent Document 2).

The platelet aggregation inhibitory action of aspirin is known to be based on the suppression of cyclooxygenase. Two cyclooxygenases, namely, cyclooxygenase-1 (COX-1) and cyclooxygenase-2 (COX-2) are known, and aspirin selectively and irreversibly inhibits COX-1 at a low dose to suppress the platelet aggregation. However, this inhibition of COX-1 is also a cause for the aspirin ulcer (see Non-Patent Documents 3 and 4).

As described above, while aspirin is useful as a platelet aggregation inhibitor, it is associated with the side effect of gastrointestinal dysfunction caused by the COX-1 inhibitory action which is the mechanism enabling the aspirin function. Accordingly, a platelet aggregation inhibitor free from the COX-1 inhibitory action is highly desired.

Nonsteroidal anti-inflammatory drugs are known to exhibit anti-inflammatory action by selective inhibition of COX-2. However, some selective COX-2 inhibitors are known to induce side effects in cardiovascular system as well as thrombosis (Non-Patent Documents 5 to 7).

Pyrazole derivatives known to have antithrombotic action include Compound (A) (see Patent Document 1 and Non-Patent Document 8) and Compound (B) (see Patent Document 2). However, inhibitory action for the collagen-induced platelet aggregation of these compounds is not strong compared to the COX inhibitory action of these compounds, and these compounds are associated with a high risk of developing a side effect like that of the aspirin.

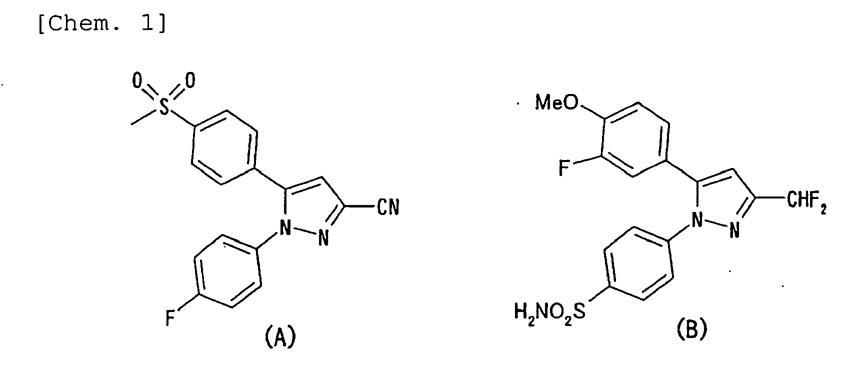

Patent Document 1: Japanese Patent No. 2586713
Patent Document 2: International Patent Publication WO97/29774
Non-Patent Document 1: BMJ, vol. 308, pages 81-106, 1994
Non-Patent Document 2: BMJ, vol. 321, pages 1183-1187, 2000
Non-Patent Document 3: Neurology, vol. 57, Suppl.2, pages S5-S7, 2001
Non-Patent Document 4: Drugs Today, vol. 35, pages 251-265, 1999
Non-Patent Document 5: N.Eng.J.Med. vol. 343, pages 1520-1528, 2000
Non-Patent Document 6: JAMA vol. 286, pages 954-959, 2001
Non-Patent Document 7: Arthritis Rheum. vol. 43, pages 1891-1896, 2000
Non-Patent Document 8: Chem.Pharm. Bull. , vol. 45, pages 987-995, 1997

### Disclosure of the Invention

### Problems to be Solved by the Invention

An object of the present invention is to provide a compound having a strong action of suppressing the platelet aggregation as well as excellent physical properties, safety, and oral absorbability. Another object of the present invention is to provide a method for producing such compound.

### Means for Solving the Problems

The inventors of the present invention have made an extensive study to find such a platelet aggregation inhibitor, and found that particular salts of pyrazole derivatives exhibit strong action of inhibiting the platelet aggregation without inhibiting COX-1and COX-2, and such platelet aggregation inhibitors have excellent properties for a drug including high oral absorbability and stability. The present invention has been achieved on the basis of such finding.

The present invention provides hydrogen chloride salt (hereinafter referred to as Compound (1b)) of [1-(6-methoxypyridazine-3-yl)-5-(pyridine-2-yl)-1H-pyrazole 3-yl](4-methylpiperazine-l-yl)methanone represented by formula (Ia):

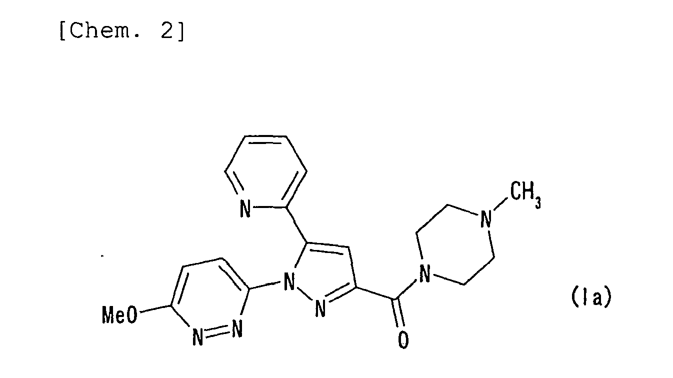

(hereinafter referred to as Compound (1a) and L- tartaric acid salt (hereinafter referred to as Compound (1c)) of Compound (1a).
The present invention also provides a method of producing such compounds.
The present invention also provides pharmaceutical compositions containing such compound and a pharmaceutically acceptable carrier.
The present invention also provides medicaments for preventing and/or treating an ischemic disease containing such compound as its effective component.
The present invention also provides methods for preventing and/or treating an ischemic disease by administering an effective amounts of such compound.
The present invention also provides use of such compounds in producing medicaments.

### Effects of the Invention

Compounds (1b) and (1c) of the present invention are capable of suppressing the platelet aggregation without inhibiting COX-1 and COX-2. Compounds (1b) and (1c) also have the action of inhibiting thrombogenesis, high crystal stability, and good oral absorbabilty. Accordingly, Compounds (1b) and (1c) of the present invention are useful in preventing and/or treating ischemic diseases caused by thrombus and embolus such as myocardial infarction, angina pectoris (chronic stable angina, unstable angina, etc.), ischemic cerebro-vascular accident (transient ischemic attack(TIA), cerebral infarction, etc.), dysfunction of peripheral arteries, occlusion after replacement of a blood vessel with an artificial blood vessel, occlusion by thrombus after the intervention of coronary artery (coronary artery bypass graft (CAGB) surgery, percutaneous transluminal coronary angioplasty (PTCA), stent indwelling, etc.), diabetic retinopathy and nephropathy, and occlusion after replacement of a cardiac valve with an artificial cardiac valve. Compounds (1b) and (1c) of the present invention are also useful in preventing and/or treating thrombus and embolus associated with vascular operations, extracorporeal blood circulation, and the like. Compounds (1b) and (1c) of the present invention are also useful in improving ischemic symptoms such as ulcer, pain, cold sensation associated with chronic artery occlusive disease.

### Brief Description of the Drawings

FIG. 1 shows the results of X-ray diffraction (FIG. 1A) and thermal analysis (TG/DTA) (FIG. 1B) for anhydride of Compound (1b).
FIG. 2 shows the results of X-ray diffraction (FIG. 2A) and thermal analysis (TG/DTA) (FIG. 2B) for dihydrate of Compound (1b).
FIG. 3 shows the results of X-ray diffraction (FIG. 3A) and thermal analysis (TG/DTA) (FIG. 3B) for anhydride of Compound (1c).
FIG. 4 shows moisture absorption and desorption behavior for Compound (1a) (FIG. 4A), Compound (1b) (FIG. 4B), and Compound (1c) (FIG. 4C).
FIG. 5 shows platelet aggregation in PRP of a guinea pig which was induced by collagen 1 hour after the oral administration of Compound (1b) .

### Best Mode for Carrying Out the Invention

The Compounds (1a), (1b), and (1c) of the present invention (respectively represented by formulae (Ia), (Ib), and (Ic)) can be produced by the production procedures as described below.

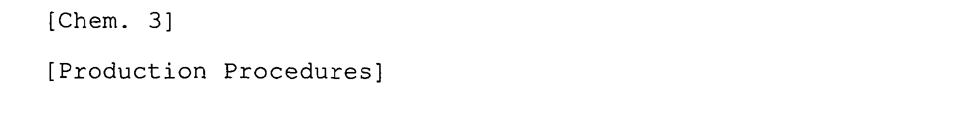

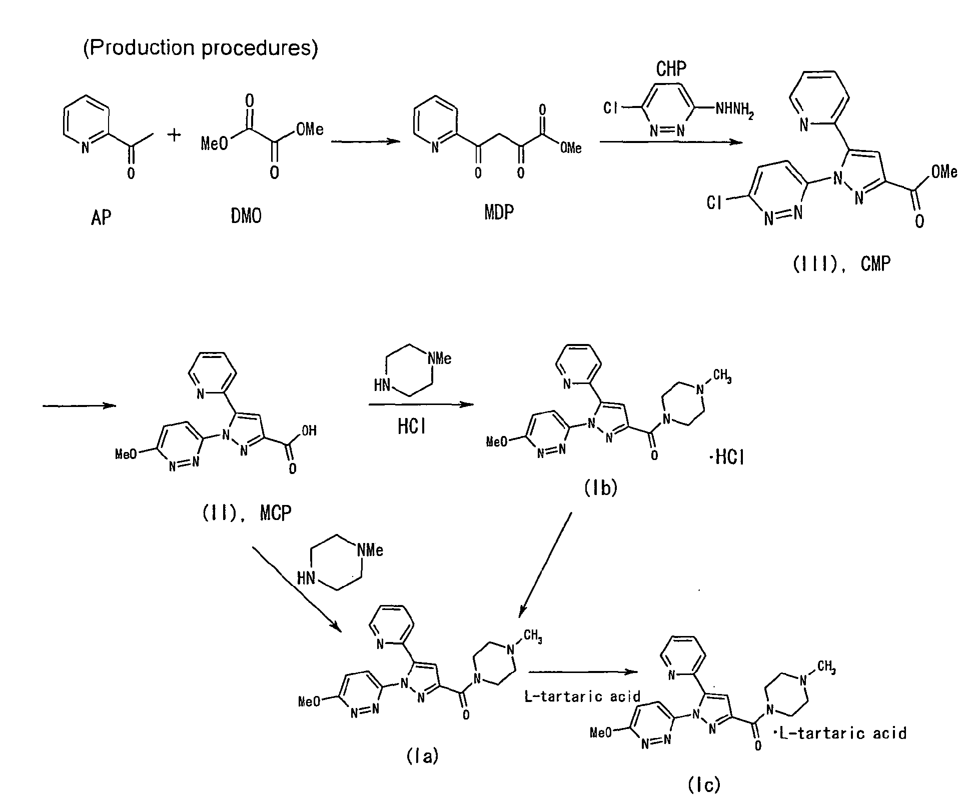

These production procedures are hereinafter described in further detail.
Acetylpyridine (AP) and dimethyl oxalate (DMO) may be treated in alcohol (methanol or ethanol) in the presence of sodium alkoxide (sodium methoxide or sodium ethoxide) to obtain methyl 2,4-dioxo-4-(2-pyridinyl)butanoate (MDP). Reaction temperature is preferably in the range of -10 to 100°C. AP and DMO used may be a commercially available or can be produced by common knowledge of organic chemistry.

Next, an equivalent amount of 3-chloro-6-hydrazinopyridazine (CHP), and then, an adequate amount of hydrochloric acid may be added to MDP in methanol at room temperature, and the mixture may be heated under reflux to produce methyl 1-(6-chloro-3-pyridazinyl)-5-(2-pyridinyl)-1H-pyrazole-3-carboxylate (CMP). The 3-chloro-6-hydrazinopyridazine used may be either a commercially obtainable or can be produced by reacting dichloropyridazine with hydrazine or similar methods.

Alternatively, after adding the equivalent amount of CHP at room temperature to MDP in methanol, an adequate amount of acid may be added and the mixture may be heated under reflux. The reaction solution may be cooled to about 40°C to 60°C, and sodium methoxide, and then, a base may be added to produce 1-(6-methoxy-3-pyridazinyl)-5-(2-pyridinyl)-IH-pyrazole-3-carboxy lic acid (MCP) represented by formula (II). Examples of the acid added include hydrochloric acid and methanesulfonic acid, and the acid is preferably hydrochloric acid. Sodium hydroxide can be used as the base. This procedure is capable of producing MCP at a high yield since purification of an intermediate such as CMP is unnecessary.

CMP can be produced into MCP in one step and at a high yield by adding sodium methoxide and a base sequentially. The base used ispreferablysodium hydroxide. The reaction temperature is preferably in the range of -20 to 100°C, and especially preferably -5 to 50°C. MCP can be crystallized by adding hydrochloric acid to the reaction solution in this step. This reaction is preferably conducted in methanol.

Next, MCP and N-methylpiperazine are condensed to produce Compound (1a) or (1b) of the present invention.
This condensation can be accomplished by the method commonly used in the peptide synthesis. Exemplary peptide synthesis methods include azide method, acid chloride method, acid anhydride method, DCC (dicyclohexylcarbodiimide) method, active ester method, carbonyldiimidazolemethod, DCC/HOBT (1-hydroxybenzotriazol) method, a method using water-soluble carbodiimide, and a method using diethyl cyanophosphate, and these methods are described in Bodanszky, M., Klausner, Y.S., and Ondetti, M.A. "Peptide Synthesis" (A Wiley-Interscience publication, New York, 1976); Pettit, G.R., "Synthetic Peptides" (Elsevier Scientific Publication Company, New York, 1976); and Japanese Society of Chemistry ed., "Lectures on Experimental Chemistry, 4th edition, vol. 22, Organic Synthesis IV" (Maruzen Publishing, 1992), and the like. The additive is preferably 1-hydroxybenzotriazolmonohydrate, and the preferable condensing agent is 1-ethyl-3-(3-dimethylaminopropyl)carbodiimide or dicyclohexylcarbodiimide. In other words, the preferred are the combination of an additive and 1-ethyl-3-(3-dimethylaminopropyl)carbodiimide and a combination of an additive and dicyclohexylcarbodiimide, for example the combination of 1-hydroxybenzotriazol monohydrate and 1-ethyl-3-(3-dimethylaminopropyl)carbodiimide and the combination of 1-hydroxybenzotriazol monohydrate and dicyclohexylcarbodiimide. Various solvents can be used in this condensation, and exemplary solvents include methanol, ethanol, isopropanol, N,N-dimethylformamide, pyridine, chloroform, methylene chloride, tetrahydrofuran, dioxane, acetonitrile, water, and mixtures of such solvents. When the combination of 1-hydroxybenzotriazol monohydrate and 1-ethyl-3-(3-dimethylaminopropyl)carbodiimide is used, the solvent is preferably water-containing ethanol, and when the combination of 1-hydroxybenzotriazol monohydrate and dicyclohexylcarbodiimide is used, the solvent is preferably acetonitrile. The reaction temperature is preferably -20 to 50°C, and more preferably -10 to 30°C. The N-methylpiperazine used may be a commercially available or can be produced by the method disclosed in references or similar methods.

Compound (1b) can be produced as crystals when concentrated hydrochloric acid is also present in the step of the condensation. In this reaction, Compound (1b) can be obtained at a high yield since purification of Compound (1a) is not necessary.

Compound (1b) can also be produced by treating Compound (1a) with hydrochloric acid. In this case, however, crystals of Compound (1a) adapted for salt formation should be produced, and the production of Compound (1a) adapted for salt formation requires a treatment as slurry using an organic solvent such as hexane which is not suitable for use in producing a medicament.

Hexane is a solvent classified in Class 2 (solvents whose residue in medicaments should be limited) in the ICH guideline, and a very strict concentration limit of 290 ppm is imposed on this solvent. On the other hand, in the case of the method of the present invention, the production can be accomplished solely by using ethanol, isopropanol, or other solvents of Class 3 (solvents with lower toxicity and lower risk to human health whose used of up to 5000 ppm is allowed). Accordingly, Compound (1b) which can be produced without using the solvent of Class 2 is quite valuable as an active pharmaceutical ingredient.

Compound (1b) of the present invention exhibits good moisture absorption and desorption behavior as well as good solubility in all of water, First solution of Japanese Pharmacopoeia (JP1 solution), and Second solution of Japanese Pharmacopoeia (JP2 solution) . Compound (1b) of the present invention also showed strong suppression of aggregation in human and guinea pig platelets. Compound (1b) also exhibited favorable oral absorbability, and it showed remarkable suppression of thrombogenesis in the thrombus model induced by iron chloride. Compound (1b) also showed no safety problems.

Compound (1a) can also be produced by treating Compound (1b) with sodium hydroxide. Accordingly, Compound (1b) is also useful as an intermediate for producing Compound (1a).

Compound (1c) can be produced by dissolving Compound (1a) in ethanol and reacting with L-tartaric acid.

Compound (1c) of the present invention does not exhibit crystal polymorphism, and it shows excellent moisture absorption and desorption behavior. Compound (1c) is also highly soluble in all of water, JP1 solution, and JP2 solution, and showed no change in crystal form at different humidity. Compound (1c) was also extremely stable under wet heat, dry heat, and beam irradiation. Accordingly, Compound (1c) of the present invention is highly qualified as an active pharmaceutical ingredient particularly in view of its physical properties.

The present invention also relates to a method for producing a compound (MCP) represented by formula (II):

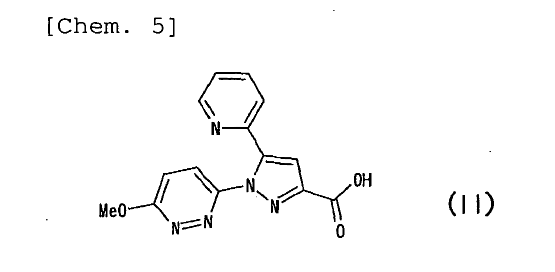

at a high yield by reacting the compound (CMP) represented by formula (III) :

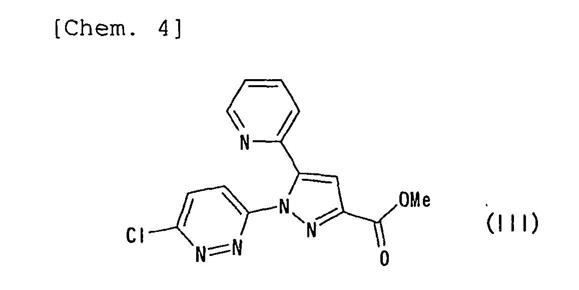

with sodium methoxide in methanol, and treating the reaction mixture with sodium hydroxide.

This method is capable of producing MCP from CMP in one step and at a high yield with no need of purifying methyl 1-(6-methoxy-3-pyridazinyl)-5-(2-pyridyl)pyrazole-3-carboxylate (MMP) . The yield of the MCP from CMP was not sufficient when MCP was produced by treating CMP with sodium methoxide-methanol solution, isolating MMP, and treating MMP with a base. The method of the present invention is capable of producing MCP in 97% yield.

The present invention also relates to a method for producing a compound represented by formula (II):

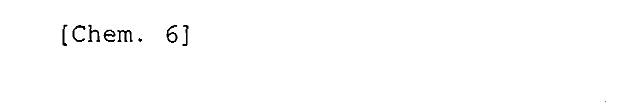

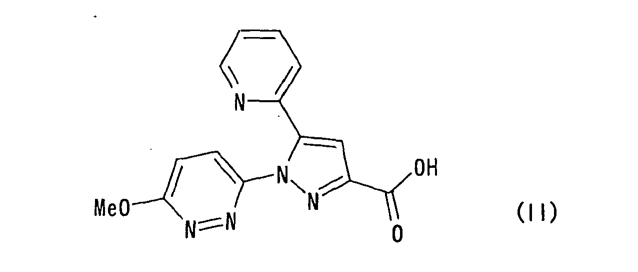

comprising the steps of reacting a compound represented by formula (IV):

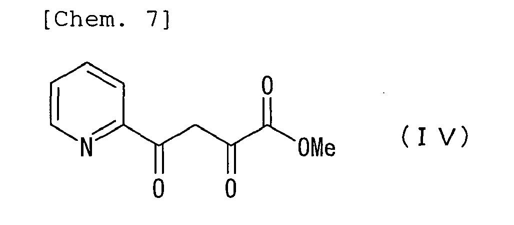

with 3-chloro-6-hydrazinopyridazine in methanol in the presence of concentrated hydrochloric acid or in the presence of methanesulfonic acid, adding sodium methoxide, and treating the reaction mixture with sodium hydroxide.
This method can produce MCP at a high yield since this method is capable of producing MCP from MDP with no need of purifying the intermediates such as CMP.

The present invention also provides a method for producing a compound represented by formula (Ic):

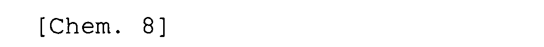

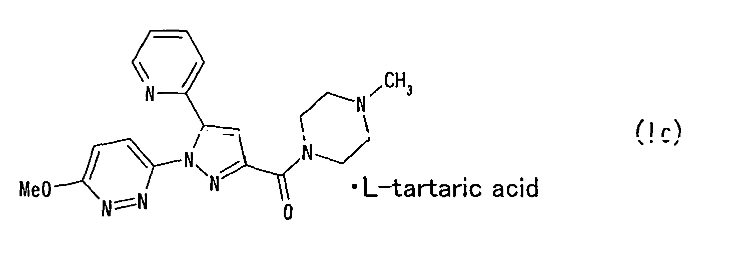

comprising the steps of reacting MCP represented by formula (II):

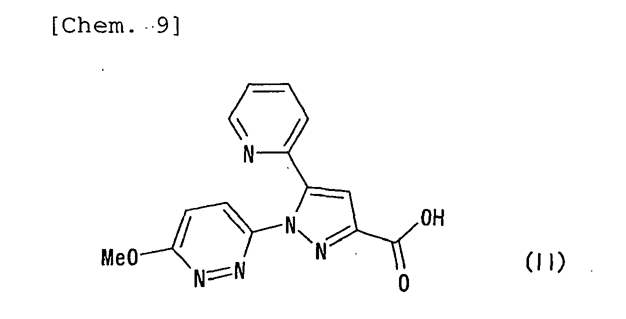

with N-methylpiperazine in acetonitrile in the presence of 1-hydroxybenzotriazol monohydrate and 1-ethyl-3-(3-dimethylaminopropyl)carbodiimide to produce a compound represented by formula (Ia):

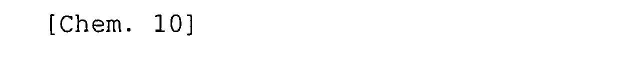

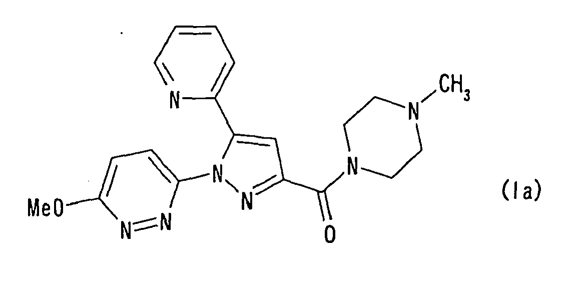

; and followed by reacting this compound with L-tartaric acid.

The present invention also provides a method for producing a compound represented by formula (Ib): 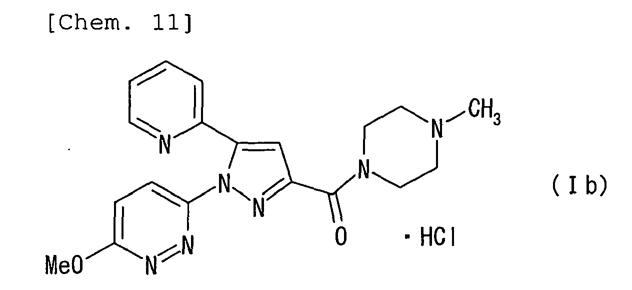 comprising the steps of reacting a compound represented by formula (II): 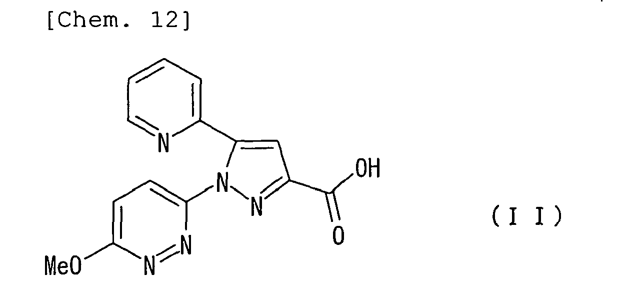 with N-methylpiperazine in acetonitrile or water-containing ethanol in the presence of 1-hydroxybenzotriazol monohydrate and 1-ethyl-3-(3-dimethylaminopropyl)carbodiimide or in the presence of 1-hydroxybenzotriazol monohydrate and dicyclohexylcarbodiimide; and treating the reaction mixture with concentrated hydrochloric acid.

The present invention also provides a method for producing a compound represented by formula (Ib): 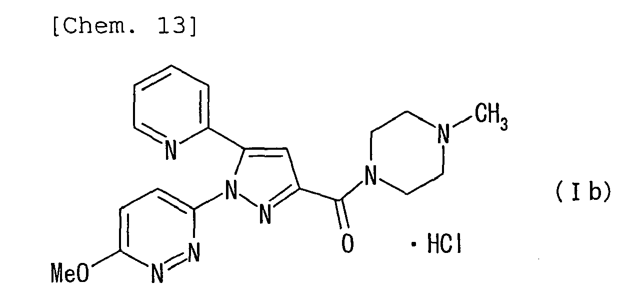 comprising the steps of reacting a compound represented by formula (III) : 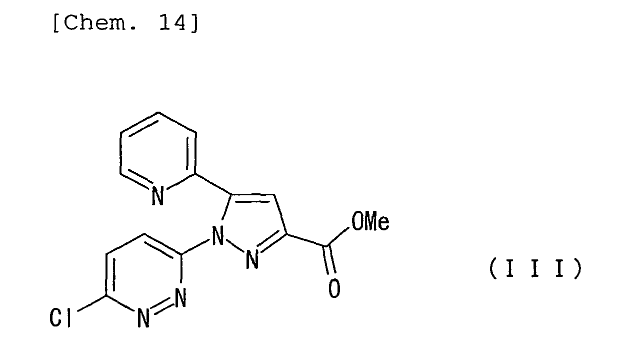 with sodium methoxide in methanol; treating the reaction mixture with sodium hydroxide to produce a compound represented by formula (II): 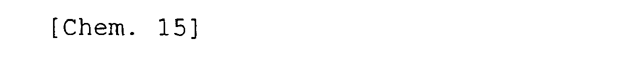 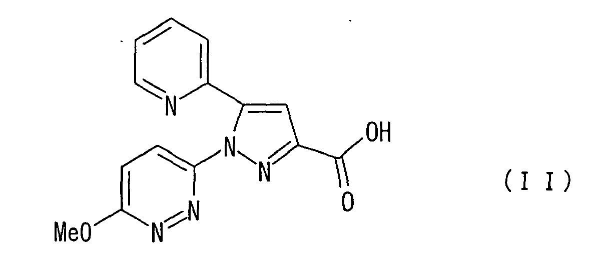 ; reacting this compound with N-methylpiperazine in acetonitrile or water-containing ethanol in the presence of 1-hydroxybenzotriazol monohydrate and 1-ethyl-3-(3-dimethylaminopropyl)carbodiimide or in the presence of 1-hydroxybenzotriazol monohydrate and dicyclohexylcarbodiimide; and treating the reaction mixture with concentrated hydrochloric acid.

The present invention also provides a method for producing a compound represented by formula (Ib): 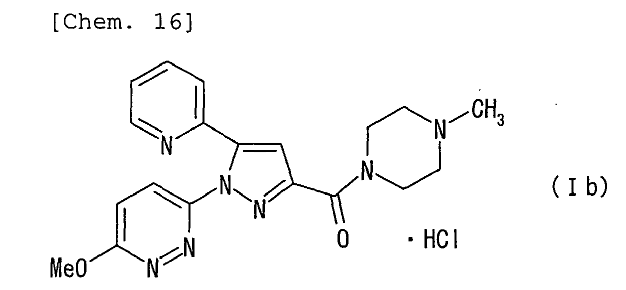 comprising the steps of reacting a compound represented by formula (IV): 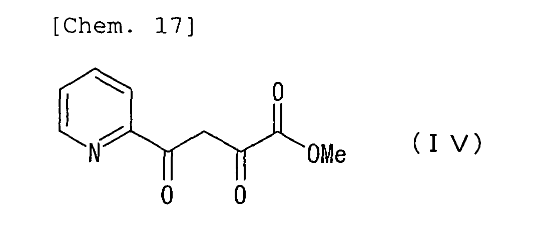 with 3-chloro-6-hydrazinopyridazine in methanol in the presence of concentrated hydrochloric acid to produce a compound represented by formula (III): 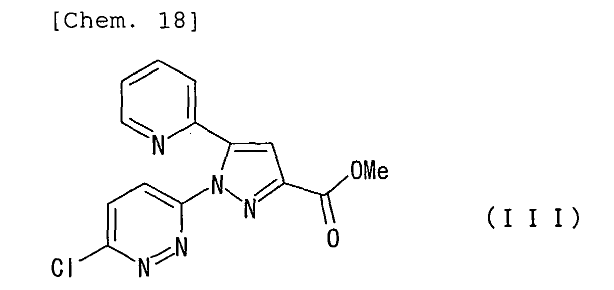 ; reacting this compound with sodium methoxide in methanol; treating the reaction mixture with sodium hydroxide to produce a compound represented by formula (II): 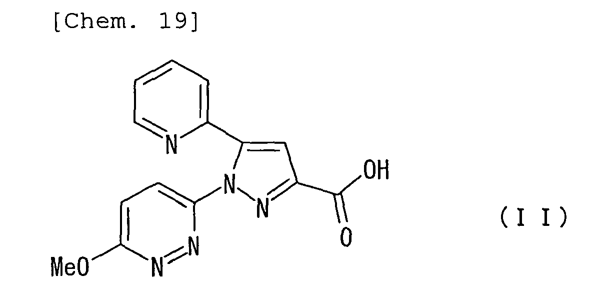 ; reacting this compound with N-methylpiperazine in acetonitrile or water-containing ethanol in the presence of 1-hydroxybenzotriazol monohydrate and 1-ethyl-3-(3-dimethylaminopropyl)carbodiimide or in the presence of 1-hydroxybenzotriazol monohydrate and dicyclohexylcarbodiimide; and treating the reaction mixture with concentrated hydrochloric acid.

The present invention also provides a method for producing a compound represented by formula (Ib): 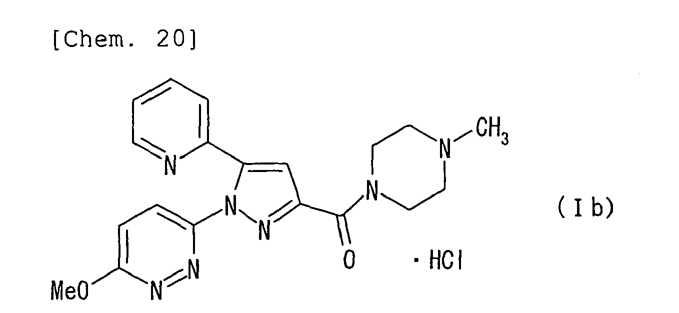 comprising the steps of treating acetylpyridine and dimethyl oxalate with methanol in the presence of sodium methoxide to produce a compound represented by formula (IV): 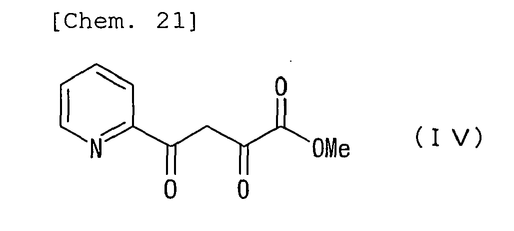 ; reacting this compound with 3-chloro-6-hydrazinopyridazine in methanol in the presence of concentrated hydrochloric acid to produce a compound represented by formula (III): 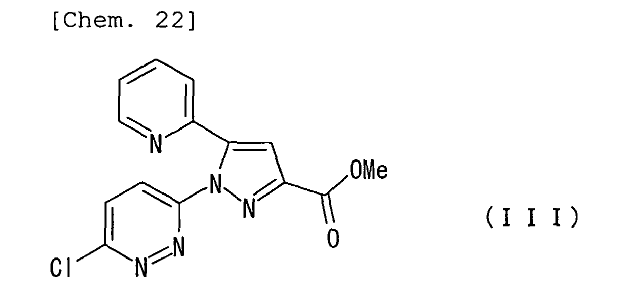 ; reacting this compound with sodium methoxide in methanol; treating this compound with sodium hydroxide to produce a compound represented by formula (II) : 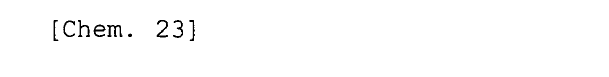 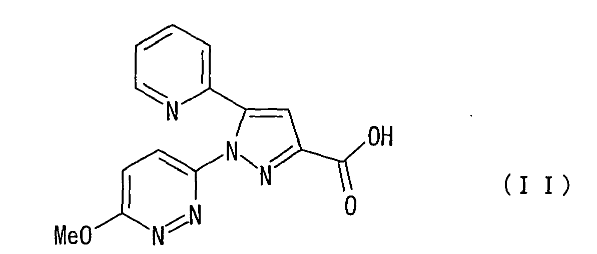 ; reacting this compound with N-methylpiperazine in acetonitrile or water-containing ethanol in the presence of 1-hydroxybenzotriazol monohydrate and 1-ethyl-3-(3-dimethylaminopropyl)carbodiimide or in the presence of 1-hydroxybenzotriazol monohydrate and dicyclohexylcarbodiimide; and treating the reaction mixture with concentrated hydrochloric acid.

The present invention also provides a method for producing a compound represented by formula (Ib):

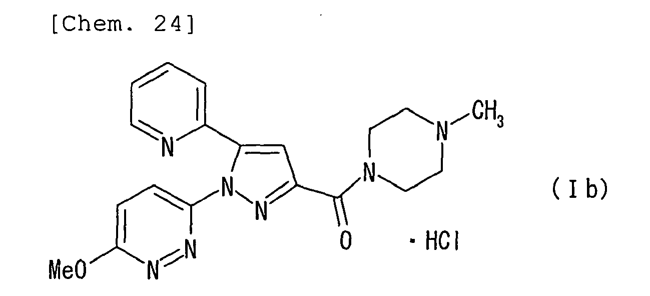

comprising the steps of reacting a compound represented by formula (IV):

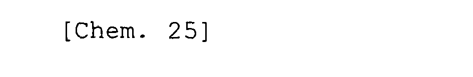

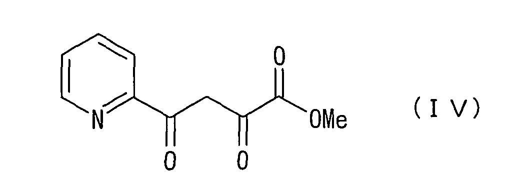

with 3-chloro-6-hydrazinopyridazine in methanol in the presence of methanesulfonic acid or in the presence of concentrated hydrochloric acid, adding sodium methoxide, and treating the reaction mixture with sodium hydroxide to produce a compound represented by formula (II):

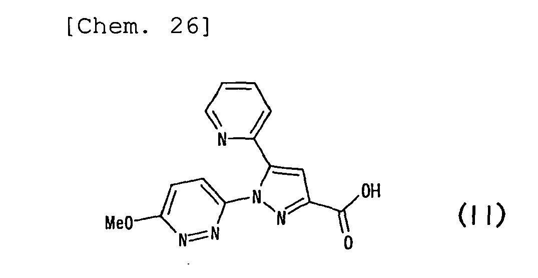

; reacting this compound with N-methylpiperazine in acetonitrile or water-containing ethanol in the presence of 1-hydroxybenzotriazol monohydrate and 1-ethyl-3-(3-dimethylaminopropyl)carbodiimide or in the presence of 1-hydroxybenzotriazol monohydrate and dicyclohexylcarbodiimide, and treating the reaction mixture with concentrated hydrochloric acid.

The present invention also provides a method for producing a compound represented by formula (Ib):

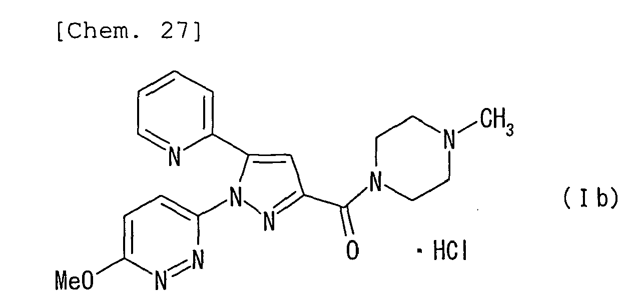

comprising the steps of treating acetylpyridine and dimethyl oxalate in methanol in the presence of sodium methoxide to produce a compound represented by formula (IV):

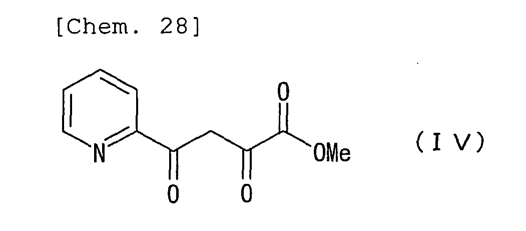

; reacting this compound with 3-chloro-6-hydrazinopyridazine in methanol in the presence of methanesulfonic acid or in the presence of concentrated hydrochloric acid; adding sodium methoxide; treating the reaction mixture with sodium hydroxide to produce a compound represented by formula (II):

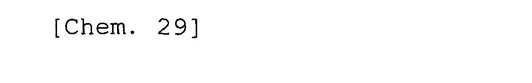

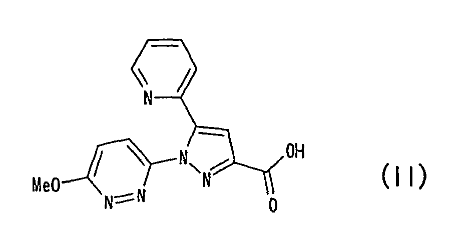

; reacting this compound with N-methylpiperazine in acetonitrile or water-containing ethanol in the presence of 1-hydroxybenzotriazol monohydrate and 1-ethyl-3-(3-dimethylaminopropyl)carbodiimide or in the presence of 1-hydroxybenzotriazol monohydrate and dicyclohexylcarbodiimide; and treating the reaction mixture with concentrated hydrochloric acid.

It has been impossible to directly synthesize Compound (1c) from MCP or to produce Compound (1c) from Compound (1b) without isolating Compound (1a). The method of the present invention has enabled to produce such Compound (1c) whose production has been extremely difficult.

All of Compounds (1a), (1b), and (1c) of the present invention exhibited remarkable inhibition of the platelet aggregation induced by collagen. In addition, Compounds (1a) and (1b) exhibited strong ex vivo inhibition of the platelet aggregation by the collagen as well as excellent oral absorption. Compound (1b) exhibited remarkable inhibition of the thrombogenesis in the model of thrombosis induced by high shear stress. Accordingly, Compound (1b) of the present invention is useful as a prophylactic and/or therapeutic agent for ischemic diseases caused by thrombus and embolus such as myocardial infarction, angina pectoris (chronic stable angina, unstable angina, etc.), ischemic cerebro-vascular accident (transient ischemic attack(TIA),cerebralinfarction,etc.),peripheralvascular disorder, occlusion after replacement of a blood vessel with an artificial blood vessel, occlusion by thrombus after the intervention of coronary artery (coronary artery bypass graft (CAGB) surgery, percutaneous transluminal coronary angioplasty (PTCA), stent indwelling, etc.), diabetic retinopathy and nephropathy, and occlusion after replacement of a cardiac valve with an artificial cardiac valve in human and other mammals. Compound (1b) of the present invention is also useful as a prophylactic and/or therapeutic agent for thrombus and embolus associated with vascular operations,extracorporealblood circulation, and the like. It is also useful in improving ischemic symptoms such as ulcer, pain, cold sensation associated with chronic artery occlusive disease.

When Compound (1a), (1b), or (1c) of the present invention is used as an active pharmaceutical ingredient, the dose of these compounds may depend on the age, sex, and symptoms of the patient. The dose is preferably in the range of 0.1 mg to 1 g, and especially preferably 0 .5 mg to 500 mg per day for an adult. This daily dose may be administered in several divided doses, and if necessary, the compounds may be administered at a dose exceeding such daily dose.

The medicaments containing Compound (1a), (1b), or (1c) of the present invention as its effective component may be used in any desired route of administration and dosage form. The medicaments may be prepared by any of the methods commonly used in the art, optionally blending the compound with any desired pharmaceutically acceptable carrier, into a dosage form matching the route of administration, and the route of administration and the dosage form are not particularly limited.
Exemplary oral preparations include solid preparations such as tablet, powder, granule, pill, and capsule as well as liquid preparations such as solution, syrup, elixir, suspension, and emulsion.
An injectable may be prepared by filling a solution of Compound (1a), (1b) or (1c) in a container, or by solidifying the solution by freeze drying for reconstitution immediately before the use.
In the preparation of the medicaments, pharmaceutically acceptable additives such as binder, disintegrant, solubilizer, lubricant, filler, and excipient may be added as desired.

### Examples

Next, the present invention is described in further detail by referring to the Examples.

### [Example 1] Synthesis of [1-(6-methoxypyridazine-3-yl)-5-(pyridine-2-yl)-1H-pyrazole-3-yl] (4-methylpiperazine-1-yl)methanone monohydrochloride (Compound (1b))

### [Example 1-1] Methyl 2,4-dioxo-4-(2-pyridinyl)butanoate (MDP)

To a 10 L four necked flask was added methanol (5.3 L), and sodium methoxide (402 g) was dissolved. Dimethyl oxalate (878 g) and acetylpyridine (600 g, 556 mL) were then added. A dropping funnel was washed with 200 mL of methanol. The reaction suspension was stirred at room temperature for 16.5 hours, and the reaction solution was moved to a stainless can. To this solution was added 11.1 L of water, and the mixture was cooled on ice. Next, 5N hydrochloric acid (10 L) was added to adjust the mixture to a pH of 3.0. The reaction mixture was stirred for 2.5 hours in an ice bath, and the crystals were collected by filtration. The thus obtained crystals were washed once with 300 mL of water, and twice with 10 L of isopropanol. The crystals were dried under reduced pressure to obtain the title compound (904 g, 88%). mp: 86-87°C; ¹H NMR (CDCl₃) δ: 3.95 (s, 3H), 7.52 (br, 1H), 7.64 (br, 1H), 7.91 (br, 1H), 8.17 (br, 1H) 8.73 (br, 1H).

### [Example 1-2] Methyl 1-(6-chloro-3-pyridazinyl)-5-(2-pyridinyl)-1H-pyrazole-3-carboxyl ate (CMP)

To 20 L four necked flask were added methanol (6.0 L), 3-chloro-6-hydrazinopyridazine (CHP, 425 g), and MDP (600 g) produced in Example 1-1, and the mixture was heated under reflux for 2 hours. 120 mL of concentrated hydrochloric acid was added and the mixture was refluxed for 11 hour. The reaction solution was cooled to room temperature, and 2.0 L of water was added. To this mixture was then added 360 mL of 5N solution of sodium hydroxide, and the mixture was adjusted to a pH of 8.5. Water (10.0 L) was added to mixture, and the mixture was stirred at room temperature for 1 hour. The precipitated crystals were separated by filtration, and washed with 1.2 L of water. The resulting crystals were treated as slurry by adding 1.2 L of methanol containing 67% of water. The crystals were separated by filtration, and dried under reduced pressure to obtain the title compound (CMP, 665 g, 73%).
mp: 164-165°C; ¹H NMR (CDCl₃) δ: 4.00 (s, 3H), 7.25 (s, 1H) , 7.27 (dd, J=4.9, 7.7 Hz, 1H), 7.64 (d, J=7.8 Hz, 1H), 7.70 (d, J=9.0 Hz, 1H), 7.79 (dd, J=7 .7, 7.8 Hz, 1H) , 8.09 (d, J=9.0 Hz, 1H) , 8.40 (d, J=4 . 9, 1H).

### [Example 1-3] 1-(6-Methoxy-3-pyridazinyl)-5-(2-pyridinyl)-1H-pyrazole-3-carboxy lic acid (MCP)

Sodium methoxide (171 g) was dissolved in 7.5 L of methanol in a 20 L four necked flask. To this solution was added CMP (500 g) produced in Example 1-2, and the mixture was stirred at 40°C for 1.5 hours. 5N aqueous solution of sodium hydroxide (250 mL) was then added, and the mixture was stirred at 40°C for 1 hour, and the reaction solution was cooled to room temperature. To this reaction solution was added 7.5 L of water and active carbon (250 g), and the mixture was stirred for 30 minutes. The active carbon was removed by filtration, and 5N hydrochloric acid (550 mL) was added to the filtrate to adjust the pH to 3.3. The reaction solution was stirred overnight at room temperature, and the precipitated crystals were collected by filtration. The crystals were washed with cold water (1.0 L) and methanol (750 mL). The crystals were then dried under reduced pressure to obtain the title compound (MCP, 455 g, 97%).
mp: 227-8°C (decomp.) ; ¹H NMR (DMSO-d₆) δ: 4.04 (s, 3H), 7.34 (dd, J=4.7, 7.6 Hz, 1H), 7.41 (s, 1H), 7.49 (d, J=9.3 Hz, 1H), 7.81 (d, J=7.8 Hz, 1H), 7.89 (dd, J=7.6, 7.8 Hz, 1H), 7.99 (d, J=9.3 Hz, 1H), 8.35 (d, J=4.7 Hz, 1H), 13.2 (br, 1H).

### [Example 1-4] [1-(6-Methoxypyridazine-3-yl)-5-(pyridine-2-yl)-1H-pyrazole-3-yl] (4-methylpiperazine-1-yl)methanone monohydrochloride (Compound (1b))

To a 5 L four necked flask was added 95% ethanol (1.0 L), and then, MCP (260 g) produced in Example 1-3, 1-hydroxybenzotriazol monohydrate (HOBt·H₂O, 201 g), N-methylpiperazine (175 g), and 1-ethyl-3-(3-dimethylaminopropyl)carbodiimide hydrochloride (water soluble carbodiimide: WsCI, 252 g), and the mixture was stirred at room temperature for 1.5 hours. Concentrated hydrochloric acid (182 mL) was added dropwise to the reaction solution, and after further adding ethanol (1.3 L), the mixture was stirred at 50°C for 1 hour. The reaction solution was gradually cooled to room temperature (at 7°C/hour), and cooled for 2 hours in an ice bath. The resulting crystals were collected by filtration, and washed with 600 mL of ethanol. The resulting crystals were dried under reduced pressure to obtain the crude title compound (344 g, 95%). The crude title compound (340 g) was added to 70% ethanol (112 mL) in a 3 L three-necked flask, and the mixture was heated to 60°C for dissolution, the solution was then filtered through a glass filter. The flask was washed with 70% ethanol (68 mL) and ethanol (500 mL), and the washing was added to the filtrate. The filtrate was moved to a 10 L four necked flask, and after adding 3.4 L of ethanol to the filtrate, the mixture was heated to 60°C and stirred for 2 hours. The mixture was gradually cooled to 50°C, and stirred for 1.5 hours to gradually precipitate crystals. The mixture was gradually cooled ice temperature, and the crystals were collected by filtration. The crystals were then washed with 700 mL of ethanol, and dried under reduced pressure to obtain the title compound (303 g, 89%).
mp: 227-228°C (decomp.) ; ¹H NMR (d₂O) δ: 2.95 (s, 3H), 3.2-3.6 (br, to 4H), 4.09 (s, 3H), 7.26 (s, 1H), 7.47 (d, J=9.4 Hz, 1H), 7.48-7. 51 (m, 1H), 7.69 (d, J=7.8 Hz, 1H), 7.91 (d, J=9.4 Hz, 1H), 7.97 (dd, J=7.8, 7.4 Hz, 1H), 8.43 (d, J=5.1 Hz, 1H).

### [Example 2] Synthesis of 1-(6-methoxypyridazine-3-yl)-5-(pyridine-2-yl)-1H-pyrazole-3-yl]( 4-methylpiperazine-1-yl)methanone (Compound (1a))

### (Method 1)

In a 1L four necked flask was placed acetonitrile (350 mL), and after adding MCP (35.0 g) produced in Example 1-3, HOBt·H₂O (37.1 g), N-methylpiperazine (23.6 g), and WsCI (30.9 g), the mixture was stirred at room temperature for 16 hours. Triethylamine (58 mL) was added to the reaction solution, and after concentrating the mixture under reduced pressure, water (200 mL), and then, 250 mL of saturated aqueous solution of sodium hydrogencarbonate was added to precipitate the crystals. The suspension including the precipitated crystals were extracted three times with chloroform (220 mL), and then washed with 220 mL aqueous solution of sodium hydrogencarbonate and with distilled water 220 mL. The organic layer was dried over sodium sulfate, concentrated to 45 g, and 450 mL of hexane was added for treatment as slurry. The crystals were then collected by filtration, and dried under reduced pressure to obtain the title compound (41.7 g, 93%). mp: 139-140°C (decomp.); ¹H NMR (CDCl₃) δ: 2.33 (s, 3H), 2.45 (m, 2H), 2.51 (m, 2H), 3.86 (m, 2H), 4.07 (m, 2H), 4.11 (s, 3H), 7.10 (s, 1H), 7.13 (d, J=9.1 Hz, 1H), 7.22 (dd, J=4.8, 7.5 Hz, 1H), 7.58 (d, J=7.8 Hz, 1H), 7.74 (dd, J=7.5, 7.8 Hz, 1H), 7.79 (d, J=9.1 Hz, 1H), 8.41 (d, J=4.8 Hz, 1H).

### (Method 2)

In a 1L three-necked flask was placed 126.7 g of Compound (1b) produced in Example 1-4, and after adding water (640 mL), the mixture was stirred at 50°C and 5N aqueous solution of NaOH (64 mL) was added dropwise. After the completion of the dropwise addition, the reaction solution was cooled to 2°C. The reaction suspension containing the precipitated crystals was filtered, and the resulting crystals were washed with water (200 mL), and dried under reduced pressure to obtain Compound (1a) (95.9 g; 83%).

### [Example 3] Synthesis of [1-(6-methoxypyridazine-3-yl)-5-(pyridine-2-yl)-1H-pyrazole-3-yl] (4-methylpiperazine-1-yl)methanone mono-1-tartrate (Compound (1c))

To a 50 mL two neck flask were added 80% isopropanol(10 mL), Compound (1a) (2.5 g) produced in Example 2, and L-tartaric acid(1.0 g), and the mixture was heated to 60°C for dissolution. Isopropanol (5 mL), and then, isopropanol (10 mL) was added to precipitate the crystals. The temperature was gradually reduced to room temperature, and then, the mixture was cooled in an ice bath to collect the crystals by filtration. The crystals were then dried under reduced pressure to obtain the title compound (3.3 g, 96%).
mp: 180-181°C (decomp.); ¹H NMR (D₂O) δ: 2.99 (s, 3H),.3.1-3.9 (m,to 8H), 4.08 (s, 3H), 4.45 (s, 2H), 7.26 (s, 1H) , 7.46 (d, J=9.4 Hz, 1H) , 7.47-7.51 (m, 1H), 7.68 (d, J=8.0 Hz, 1H), 7.90 (d, J=9.4 Hz, 1H), 7.97 (dd, J=7.6, 8.0 Hz, 1H), 8.42 (d, J=4.9 Hz, 1H).

In the NMR spectrum of Example 1-3, a) means that TMS was used for the standard, and b) means that TPS (sodium (3-trimethylsilyl)-2,2,3,3-d4-propionate) was used for the standard.

### [Example 4] Crystals of anhydrous [1-(6-methoxypyridazine-3-yl)-5-(pyridine-2-yl)-1H-pyrazole-3-yl] (4-methylpiperazine-1-yl)methanone monohydrochloride (Compound (1b))

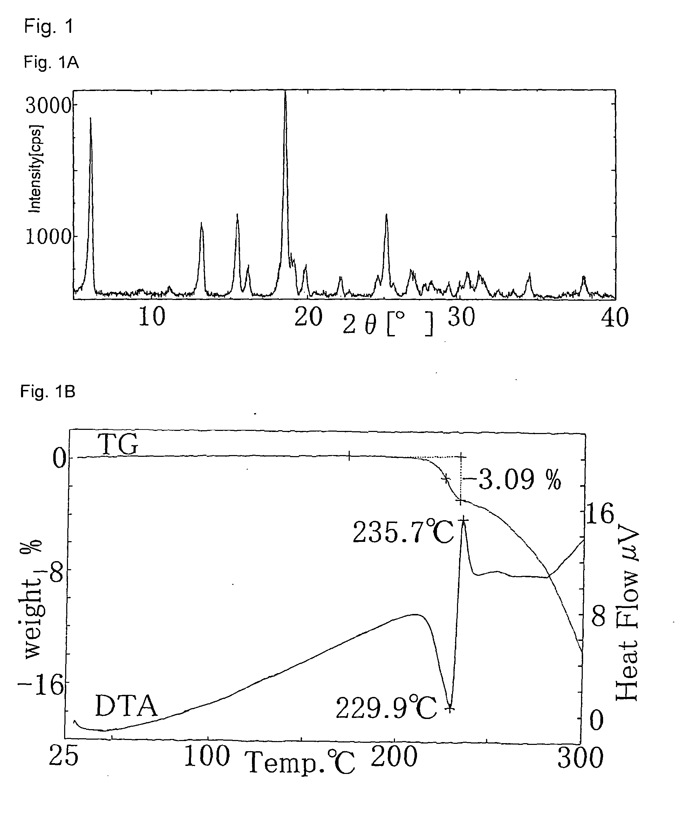

MCP was dissolved in N,N-dimethylformamide, and the solution was reacted with N-methylpiperazine in the presence of 1-hydroxybenzotriazol, 1-(3-dimethylaminopropyl)-3-ethylcarbodiimide hydrochloride, and triethylamine. The reaction solution was separated by adding water and ethyl acetate, and the aqueous layer was extracted with ethyl acetate. The organic layers were combined, washed with saturated sodium chloride solution, and dried over anhydrous sodium sulfate. After separation by filtration, and removal of the solvent under reduced pressure, the residue was purified by silica gel column chromatography to obtain Compound (1a). The resulting Compound (1a) (99.98 mg) was dissolved in ethanol containing 10% water (0.8 ml), and after adding 0.022 mL of concentrated hydrochloric acid, the mixture was heated to 70°C for dissolution. After cooling to room temperature, the reaction mixture was allowed to stand for 2 days to precipitate the crystals. The precipitated crystals were collected by filtration and dried with air to obtain 63.4 mg (58%) of the crystals of the anhydrous Compound (1b) . Elementary analysis: Calculated value (C₁₉H₂₁N₇O₂·HCl) ; C, 54.87; H, 5. 33; N, 23.57; Cl, 8.52. Measured value; C, 54.63; H, 5.33; N, 23.50; Cl, 8.64.
The crystals were evaluated by powder X-ray diffraction and thermal analysis (TG/DTA) according to the procedure commonly used in the art. The results are shown in FIGS. 1A and 1B.

### [Example 5] Crystals of [1-(6-methoxypyridazine-3-yl)-5-(pyridine-2-yl)-1H-pyrazole-3-yl] (4-methylpiperazine-1-yl)methanone monohydrochloride (Compound (1b)) dihydrate

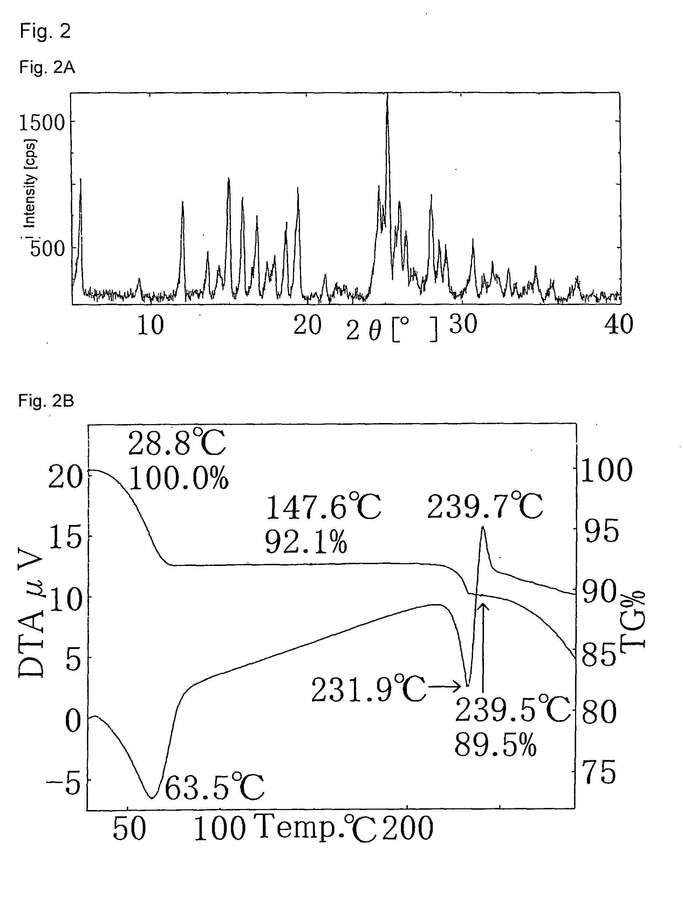

The anhydrous Compound (1b) (200.0 mg) produced in Example 1-4 was placed on a dish, and placed in a desiccator with saturated aqueous solution of potassium nitrate (93% RH) at room temperature for 7 days to thereby obtain desiccated crystals (216.1 mg).
Elementary analysis: Calculated value (C₁₉H₂₁N₇O₂·HCl·₂H₂O); C, 50.50; H, 5.80; N, 21.70; Cl, 7.85. Measured value; C, 50.42; H, 5.74; N, 21.44; Cl, 7.83.
The crystals were evaluated by powder X-ray diffraction and thermal analysis (TG/DTA) according to the procedure commonly used in the art. The results are shown in FIGS. 2A and 2B.

### [Example 6] Crystals of anhydrous [1-(6-methoxypyridazine-3-yl)-5-(pyridine-2-yl)-1H-pyrazole-3-yl] (4-methylpiperazine-1-yl)methanone mono-1-tartrate (Compound (1c))

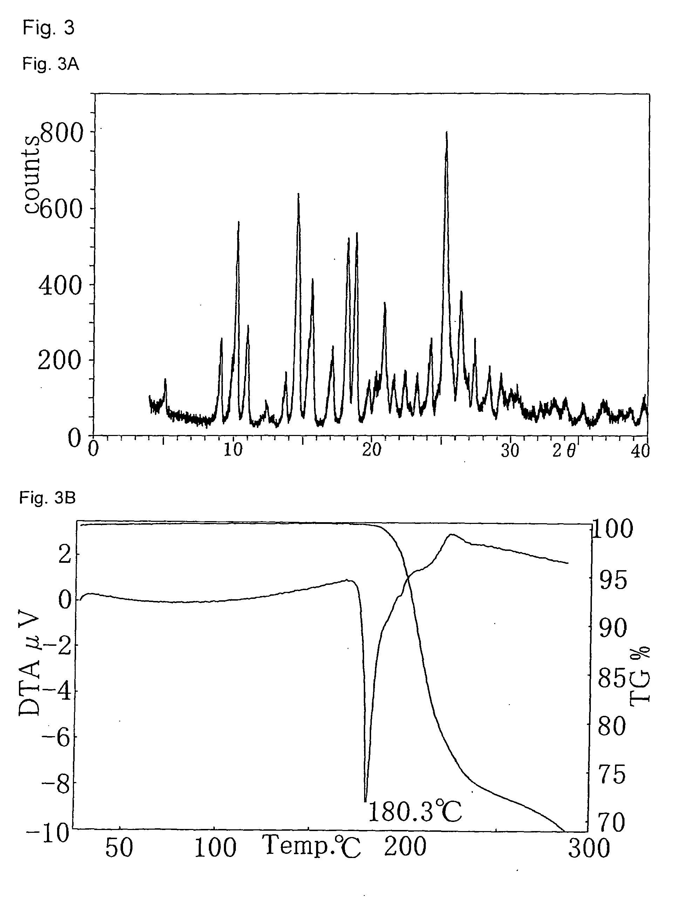

Compound (1a) (3.0 g) produced in Example 2 was dissolved in ethanol containing 5% water (30ml) , and after adding 1.24 gofL-tartaric acid, the mixture was heated to 50°C for dissolution. After cooling to room temperature, the mixture was stirred with a stirrer for 15 hours. The precipitated crystals were collected and dried with air to obtain crystals of Compound (1c) (4.0 g, 96%).
Elementary analysis: Calculated value (C₁₉H₂₁N₇O₂·C₄H₆O₆); C, 52.17; H, 5.14; N, 18.52. Measured value; C, 51.91; H, 4.95; N, 18.56.
The crystals were evaluated by powder X-ray diffraction and thermal analysis (TG/DTA) according to the procedure commonly used in the art. The results are shown in FIGS. 3A and 3B.

### [Example 7] 1-(6-Methoxy-3-pyridazinyl)-5-(2-pyridinyl)-1H-pyrazole-3-carboxy lic acid (MCP)

To a solution (100 ml) of CHP (3.5 g) in methanol were added MDP (5.0 g) and concentrated hydrochloric acid (1 ml), and the mixture was heated under reflux for 14 hours. The reaction solution was cooled to 50°C, and 28% solution (12.0 ml) of sodium methoxide in methanol was added at the same temperature. The mixture was stirred for 1 hour. 1N aqueous solution of sodium hydroxide (15.0 ml) was added to the reaction solution, and the mixture was stirred at 50°C for 1 hour and cooled to room temperature. To the reaction solution were added water (45 ml) and active carbon (250 mg), and the mixture was stirred at room temperature for 1 hour. After removing active carbon by filtration, 5N hydrochloric acid (8.0 ml) was added to the filtrate to adjust the solution to a pH of about 3.0. The solution was stirred at room temperature for another 1 hour. The precipitated crystals were collected by filtration, and dried under reduced pressure to obtain the title compound (5.73 g). Various spectroscopic data obtained for this compound were completely consistent with those of Example 1-3.

### [Example 8] 1-(6-Methoxy-3-pyridazinyl)-5-(2-pyridinyl)-1H-pyrazole-3-carboxy lic acid (MCP)

To a solution (100 ml) of CHP (3.5 g) in methanol were added MDP (5.0 g) and methanesulfonic acid (0.78 ml), and the mixture was heated under reflux for 14 hours. The reaction solution was cooled to 50°C, and 28% solution (12.0 ml) of sodium methoxide in methanol was added at the same temperature. The mixture was stirred for 1 hour. 1N aqueous solution of sodium hydroxide (15.0 ml) was added to the reaction solution, and the mixture was stirred at 50°C for 1 hour and cooled to room temperature. To the reaction solution were added water (45 ml) and active carbon (250 mg), and the mixture was stirred at room temperature for another 1 hour. After removing active carbon by filtration, 5N hydrochloric acid (8.0 ml) was added to the filtrate to adjust the solution to a pH of about 3.0. The solution was stirred at room temperature for another 1 hour. The precipitated crystals were collected by filtration, and dried under reducedpressure to obtain the title compound (5.57 g). Various spectroscopic data obtained for this compound were completely consistent with those of Example 1-3.

### [Example 9] [1-(6-Methoxypyridazine-3-yl)-5-(pyridine-2-yl)-1H-pyrazole-3-yl] (4-methylpiperazine-1-yl)methanone monohydrochloride (Compound (1b))

MCP (5.0 g) was suspended in acetonitrile (50 ml), and 1-hydroxybenzotriazol monohydrate (HOBt·H₂O, 3.4 g), N-methylpiperazine (3.7 ml), and dicyclohexylcarbodiimide (DCC, 5.2 g) were added, and the mixture was stirred at 40°C for 3 hours. Water was added to the reaction solution , and the mixture was stirred at the same temperature for 1 hour. The mixture was then cooled to room temperature, and the precipitated crystals were separated by filtration. The resulting filtrate was concentrated under reduced pressure, and the residue was dissolved in ethanol (50 ml). Concentrated hydrochloric acid (2.8 ml) was added, and the mixture was stirred at room temperature for 1 hour. The resulting crystals were collected by filtration, and dried under reduced pressure to obtain the title compound (6.3 g). Various spectroscopic data obtained for this compound were completely consistent with those of Example 1-4.

### [Test Example 1] Evaluation of moisture adsorbing and desorbing behavior

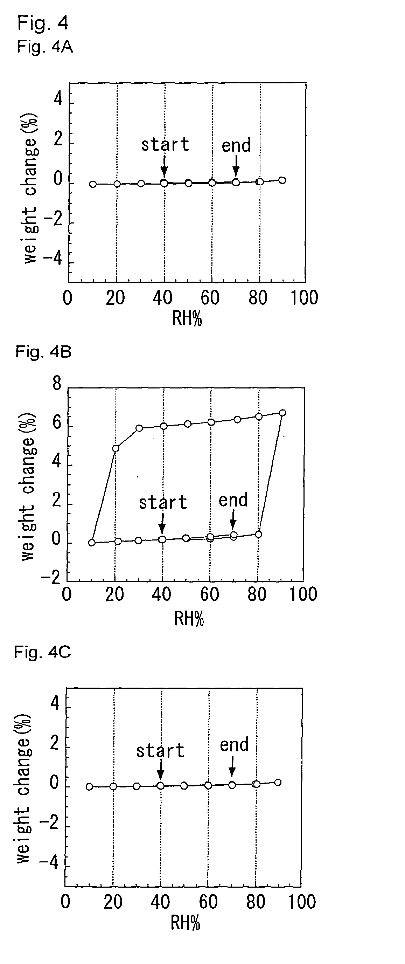

The Compounds (1a), (1b), and (1c) produced in the Examples as described above were evaluated for their moisture adsorbing and desorbing capacity by the method commonly used in the art. More specifically, about 20 mg of the crystals were placed in a Microbalance (an automatic vapor adsorption system), and change in weight with lapse of time was monitored at a relative humidity in the range of 10 to 90% to evaluate the water absorption and desorption.
It was then found that all of Compounds (1a), (1b), and (1c) exhibited weight change of only up to ±1% at the RH in the range of 40 to 60% (FIGS. 4A, 4B, and 4C).

### [Test Example 2] Evaluation of crystals for their stability

Compounds (1a), (1b), and (1c) as well as various salts of Compound (1a) produced in the Examples as described above were stored at 25°C and at a relative humidity of 0%, 52%, or 93% for 3 days to evaluate change in weight and crystal form. The results are shown in Table 1. Change in crystal form was measured in the case of fumarate at a low humidity, and in the case of Compound (1a) and its citrate at a high humidity. The salts of Compound (1a) were produced according to the method of Example 3. Compound (1c) was demonstrated to be highly stable since it showed only slight change in weight with no change in the crystal form.

**Table 1**

| | 0% RH | | 52% RH | | 93% RH | |
|---|---|---|---|---|---|---|
| | Weight change (%) | Crystal form | Weight change (%) | Crystal form | Weight change (%) | Crystal form |
| Compound (1a) | -0.41 | No¹⁾ | 0.41 | No | 7.99 | Yes |
| Compound (1b) | 0.20 | No | 0.58 | No | 11.54 | Yes |
| Compound (1c) | -0.20 | No | 0.00 | No | 0.19 | No |
| Maleate | 0.00 | No | 0.40 | No | 0.39 | No |
| Citrate | 0.00 | No | 0.00 | No | 2.45 | Yes |
| Succinate | 0.00 | No | 0.97 | No | 1.07 | No |
| Fumarate | -5.65 | Yes²⁾ | 1.60 | No | 0.59 | No |

| | | | | | | |
|---|---|---|---|---|---|---|
| 1) No: No change, 2) Yes: Some change | | | | | | |

### [Test Example 3] platelet aggregation suppression

Venous blood was collected from human and anesthetized guinea pig by using 1/10 volume of 3.13% sodium citrate for the anticoagulant, and the collected blood was centrifuged at 180 g for 10 minutes to separate platelet rich plasma (PRP) . After removing the PRP in the upper layer, the lower layer was further centrifuged at 1600 g for 10 minutes to separate the platelet poor plasma (PPP) . 1 µL of the solution of Compound (1) or Comparative Examples 1 to 3 was added to 200 µL of PRP, and the mixture was allowed to stand at 37°C for 2 minutes. Platelet aggregation was then induced by adding 2 µL of collagen to the mixture. Platelet aggregation rate was measured by using PAM-12C (SSR Engineering) . Platelet aggregation rate in the presence of each compound at each concentration was determined by using light transmittance for the PPP as the value of 100% aggregation to thereby calculate the IC₅₀ value for each compound. The results are shown in Table 2.

### [Test Example 4] Inhibition of Cyclooxygenase-1 (COX-1) and cyclooxygenase-2 (COX-2)

Screening assay kit for COX inhibitor (Cayman Chemical Company, Catalog Nos. 560101 and 560121) were used to measure inhibitory activity of each compound for COX-1 and COX-2.
Reaction buffer, heme, arachidonic acid, SnCl₂, EIA buffer, wash buffer, prostaglandin (PG) screening EIA standard, PG screening acetylcholinesterase (AchE) tracer (conjugate of a chromogenic enzyme and HRP), and PG screening EIA antiserum were prepared before the measurement.
(1) Production of PGF₂α by COX-1 or COX-2
   The reaction solution containing various concentration of the compound of the Examples and COX-1 or COX-2 was allowed to stand at 37°C for 10 minutes. 10 µL of arachidonic acid was added, and the reaction solution was allowed to stand at 37°C for 2 minutes. After the reaction, 50 µL of 1N hydrochloric acid was added to cease the reaction, and 100 µL of SnCl₂ solution was added, and the reaction solution was allowed to stand at room temperature for 5 minutes.
(2) Quantitative determination of PGF₂α by ELISA
   50 µL of an antiserum (rabbit anti-PGF₂α antibody) was added to each well of a 96 well plate which had been coated with mouse anti-rabbit IgG, and to each well was added 50 µL of a 2000-fold dilution of the PGF₂α producing reaction solution, and then, 50 µL of the AchE tracer. The reaction mixture was allowed to stand at room temperature for 18 hours. The wells were washed 5 times with the wash buffer to remove the excessive AchE tracer, and 200 µL of Ellman reagent was added. After allowing to stand in a dark room for 60 minutes, the absorbance was measured at 405 nm.

(3) Calculation of inhibitory activity
Standard curve was depicted by using PG screening EIA standard, and amount of the PGF₂α produced was determined from the absorbance. The inhibitory rate of each compound of the Examples at each concentration was determined in terms of IC₅₀ by using the amount PGF₂α produced for the reaction solution not containing the compounds of the Examples as 100%. The results are shown in Table 2.

**Table 2**

| | Suppression of aggregation by collagen (IC₅₀, µM) | | Inhibition of COX (IC₅₀, µM) | |
|---|---|---|---|---|
| | Human | Guinea pig | COX-1 | COX-2 |
| Compound (1b) (Example 1-4) | 0.86 | ND | >100 | >100 |
| Compound (1a) (Example 2) | 1.1-1.3 | 0.13 | 97 | >50 |

| | | | | |
|---|---|---|---|---|
| ND: Not Determined | | | | |

Compounds (1a) and (1b) inhibited the platelet aggregation in human and guinea pig at a low concentration. On the other hand, they exhibited IC₅₀ for COX-1 and COX-2 which is 100 fold higher than that of platelet aggregation suppression.

### [Test Example 5] Evaluation of oral absorption

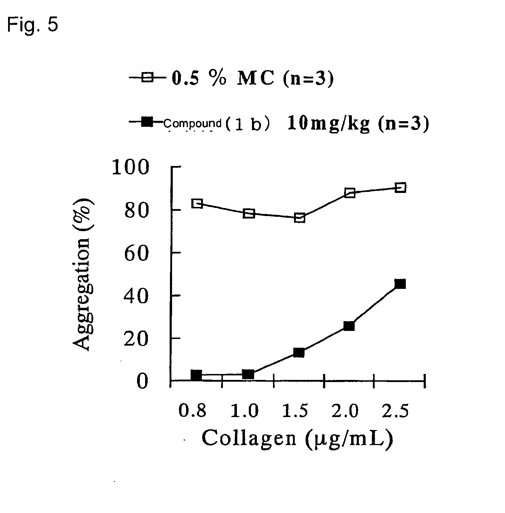

Fasted guinea pigs having a body weight of 290 to 360 g were administered with a suspension of Compound (1b) in 0.5% methylcellulose (MC) at a concentration of 2 mg/mL in terms of Compound (1a), at a dose of 10 mg/kg orally. Contrast group was solely administered with 5 mL/kg of the 0.5% MC which is the solvent for the test compound. At 1 hour after the administration, the guinea pig was anesthetized with ketamine and xylazine, and after abdominal section, blood was collected from abdominal aorta by using 1/10 volume of 3.13% sodium citrate. PRP was prepared from the thus collected blood by the same procedure as Test Example 3 to measure the platelet aggregation induced by collagen. In the platelet aggregation, the collagen was used at a final concentration of 0.8 µg to 2.5 µg.
The results are shown in FIG. 5. In the blood of the guinea pig administered with Compound (1b) at a dose of 10 mg/kg, the platelet aggregation induced by the collagen was remarkably suppressed to demonstrate that Compound (1b) is sufficiently absorbed to exhibit its effect.

## Claims

1. A compound represented by formula (Ib):

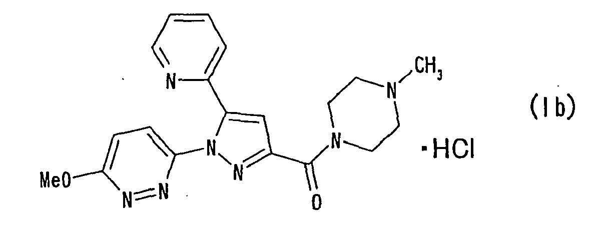

2. [1-(6-Methoxypyridazine-3-yl)-5-(pyridine-2-yl)-1H-pyrazole -3-yl](4-methylpiperazine-1-yl)methanone monohydrochloride.

3. The compound according to claim 1 or 2 wherein the compound is an anhydride.

4. The compound according to claim 1 or 2 wherein the compound is a dihydrate.

5. A compound represented by formula (Ic):

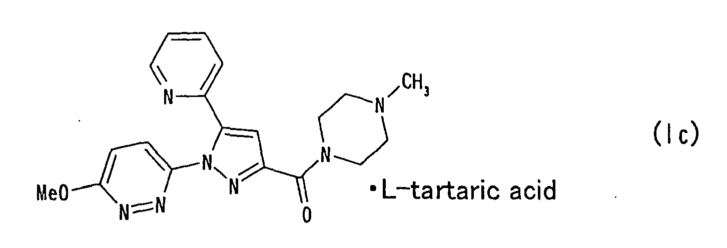

6. [1-(6-Methoxypyridazine-3-yl)-5-(pyridine-2-yl)-1H-pyrazole -3-yl](4-methylpiperazine-1-yl)methanone mono-L-tartrate.

7. The compound according to claim 5 or 6 wherein the compound is an anhydride.

8. A method for producing a compound represented by formula (Ib): 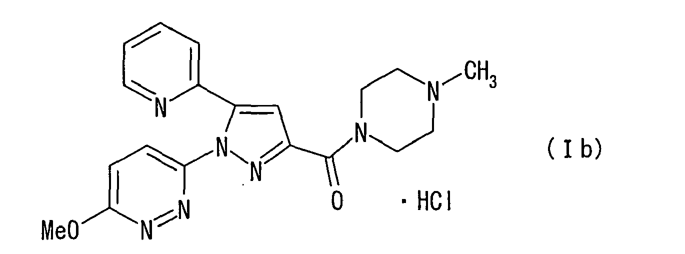 comprising the steps of reacting a compound represented by formula (II): 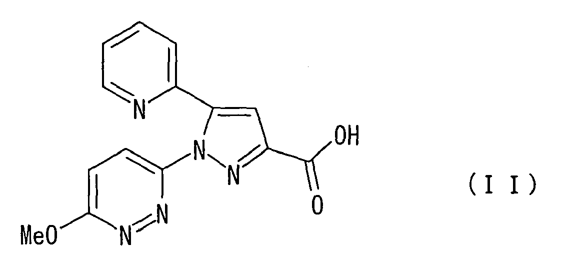 with N-methylpiperazine in water-containing ethanol in the presence of 1-hydroxybenzotriazol monohydrate and
1-ethyl-3-(3-dimethylaminopropyl)carbodiimide; and treating the reaction mixture with concentrated hydrochloric acid.

9. A method for producing a compound represented by formula (Ib) : 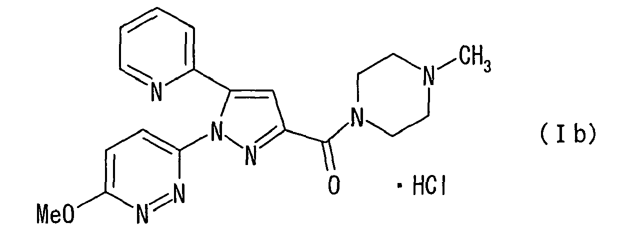 comprising the steps of reacting a compound represented by formula (III) : 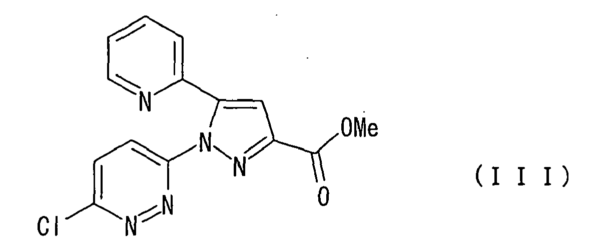 with sodium methoxide in methanol; treating the reaction mixture with sodium hydroxide to produce a compound represented by formula (II): 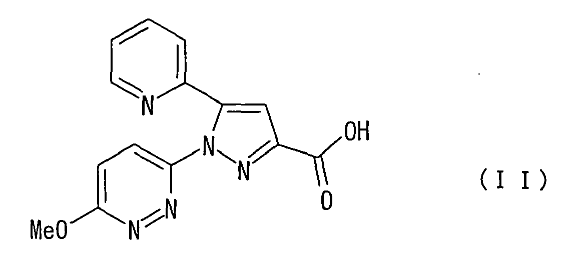 ; reacting this compound with N-methylpiperazine in water-containing ethanol in the presence of 1-hydroxybenzotriazol monohydrate and 1-ethyl-3-(3-dimethylaminopropyl) carbodiimide; and treating the reaction mixture with concentrated hydrochloric acid.

10. A method for producing a compound represented by formula (Ib) : 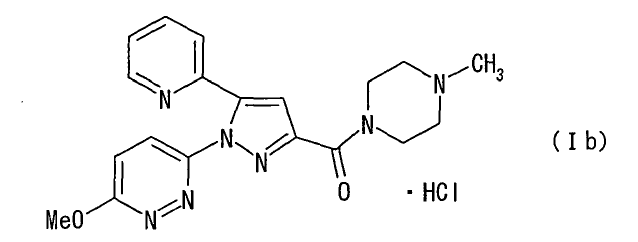 comprising the steps of reacting a compound represented by formula (IV) : 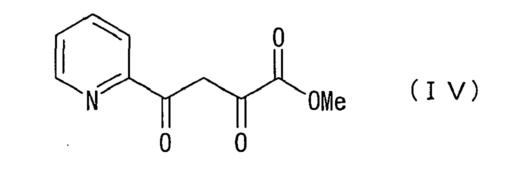 with 3-chloro-6-hydrazinopyridazine in methanol in the presence of concentrated hydrochloric acid to produce a compound represented by formula (III): 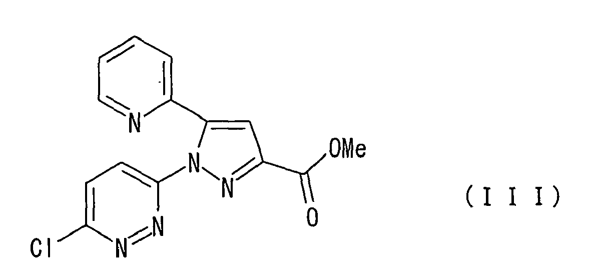 ; reacting this compound with sodium methoxide in methanol and treating the reaction product with sodium hydroxide to produce a compound represented by formula (II): 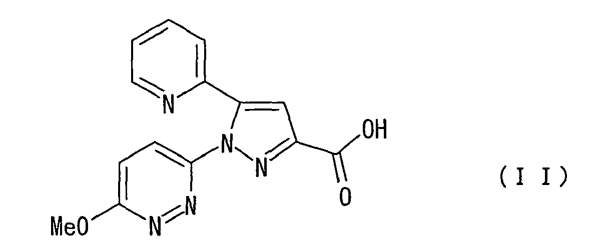 ; and reacting this compound with N-methylpiperazine in water-containing ethanol in the presence of 1-hydroxybenzotriazol monohydrate and 1-ethyl-3-(3-dimethylaminopropyl)carbodiimide; and treating the reaction mixture with concentrated hydrochloric acid.

11. A method for producing a compound represented by formula (Ib) : 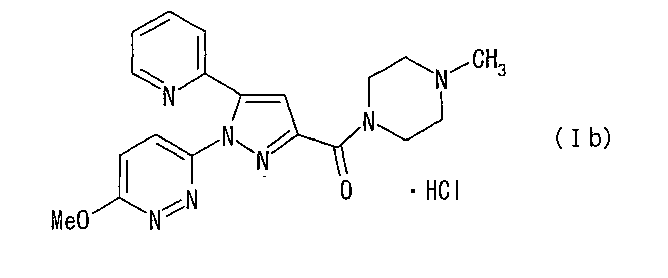 comprising the steps of reacting acetylpyridine with dimethyl oxalate in methanol in the presence of sodium methoxide to produce a compound represented by formula (IV): 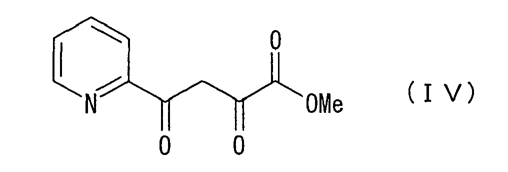 ; reacting this compound with 3-chloro-6-hydrazinopyridazine in methanol in the presence of concentrated hydrochloric acid to produce a compound represented by formula (III): 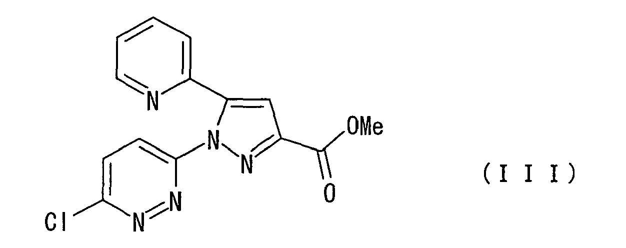 ; reacting this compound with sodium methoxide in methanol; treating the reaction mixture with sodium hydroxide to produce a compound represented by formula (II): 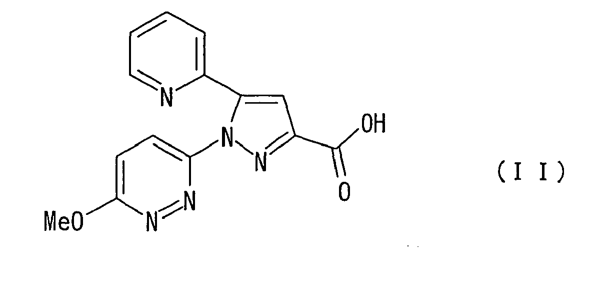 ; reacting this compound with N-methylpiperazine in water-containing ethanol in the presence of 1-hydroxybenzotriazol monohydrate and 1-ethyl-3-(3-dimethylaminopropyl) carbodiimide; and treating the reaction mixture with concentrated hydrochloric acid.

12. A method for producing a compound represented by formula (Ib) : 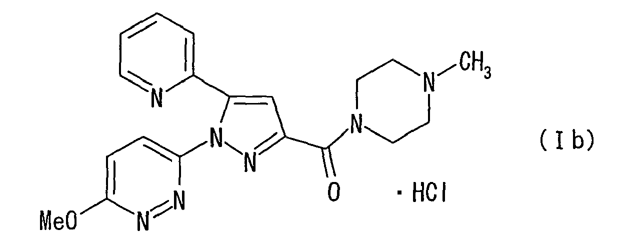 comprising the steps of reacting a compound represented by formula (IV): 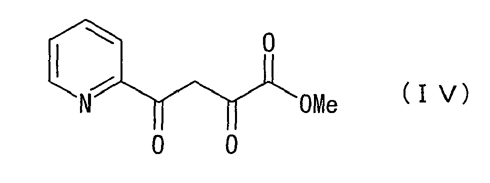 with 3-chloro-6-hydrazinopyridazine in methanol in the presence of methanesulfonic acid or in the presence of concentrated hydrochloric acid; adding sodium methoxide; and treating the reaction mixture with sodium hydroxide to produce a compound represented by formula (II): 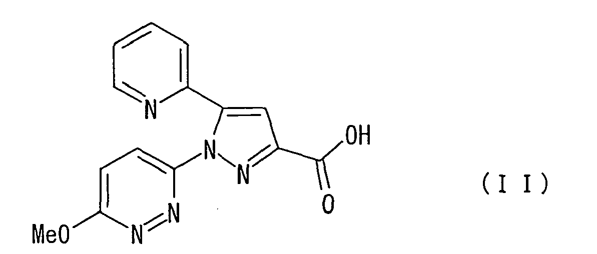 ; and reacting this compound with N-methylpiperazine in water-containing ethanol in the presence of 1-hydroxybenzotriazol monohydrate and 1-ethyl-3-(3-dimethylaminopropyl)carbodiimide; and treating the reaction mixture with concentrated hydrochloric acid.

13. A method for producing a compound represented by formula (Ib): 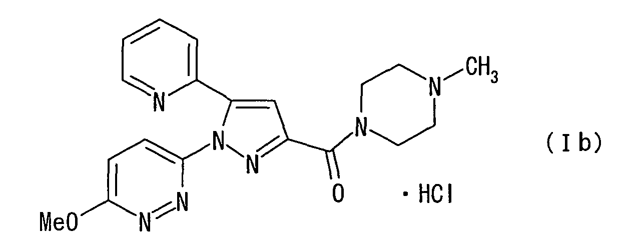 comprising the steps of reacting acetylpyridine with dimethyl oxalate in methanol in the presence of sodium methoxide to produce a compound represented by formula (IV): 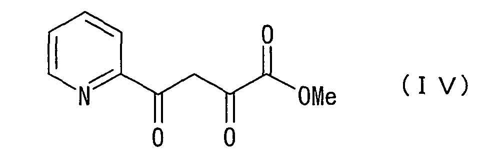 ; reacting this compound with 3-chloro-6-hydrazinopyridazine in methanol in the presence of methanesulfonic acid or in the presence of concentrated hydrochloric acid; adding sodium methoxide; treating the reaction mixture with sodium hydroxide to produce a compound represented by formula (II): 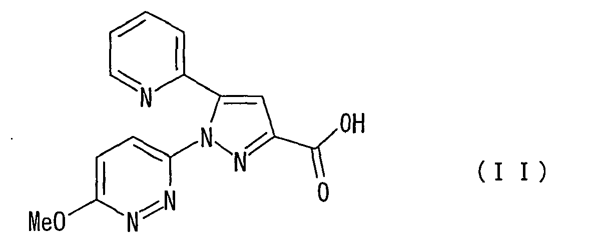 ; and reacting this compound with N-methylpiperazine in water-containing ethanol in the presence of 1-hydroxybenzotriazol monohydrate and 1-ethyl-3-(3-dimethylaminopropyl)carbodiimide; and treating the reaction mixture with concentrated hydrochloric acid.

14. A method for producing a compound represented by formula (Ib): 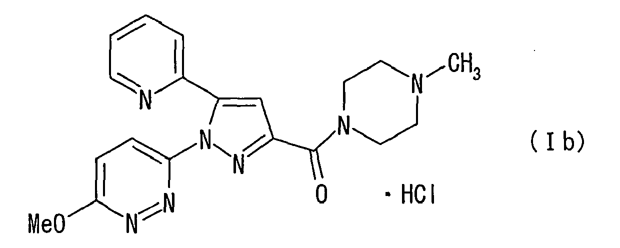 comprising the steps of reacting a compound represented by formula (II): 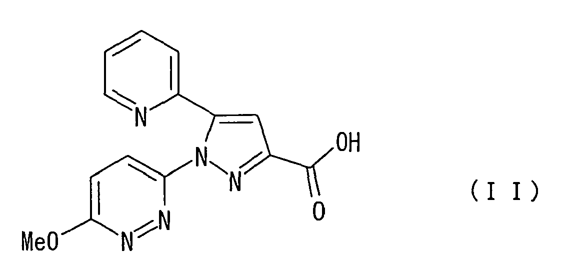 with N-methylpiperazine in acetonitrile in the presence of 1-hydroxybenzotriazol monohydrate and dicyclohexylcarbodiimide; and treating the reaction mixture with concentrated hydrochloric acid.

15. A method for producing a compound represented by formula (Ib): 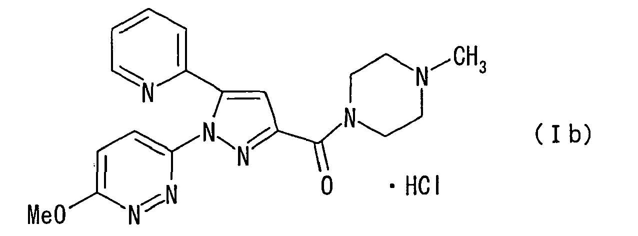 comprising the steps of reacting a compound represented by formula (III): 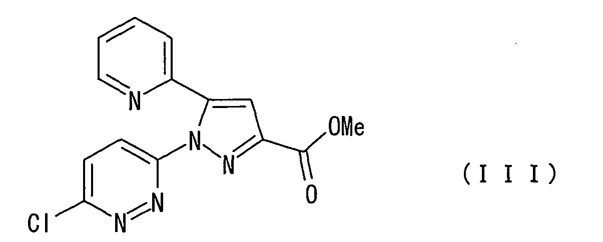 with sodium methoxide in methanol; treating the reaction mixture with sodium hydroxide to produce a compound represented by formula (II): 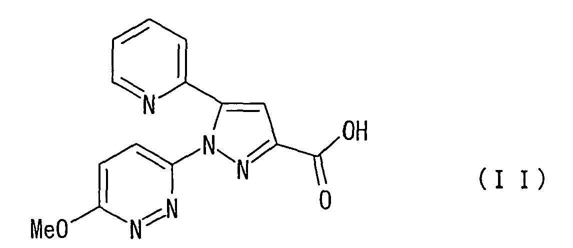 ; reacting this compound with N-methylpiperazine in acetonitrile in the presence of 1-hydroxybenzotriazol monohydrate and dicyclohexylcarbodiimide; and treating the reaction mixture with concentrated hydrochloric acid.

16. A method for producing a compound represented by formula (Ib): 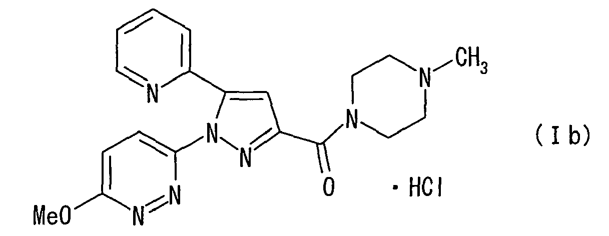 comprising the steps of reacting a compound represented by formula (IV) : 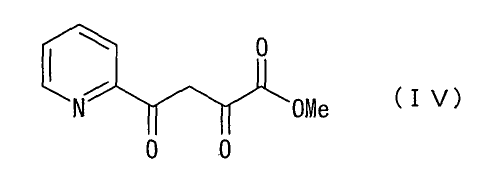 with 3-chloro-6-hydrazinopyridazine in methanol in the presence of concentrated hydrochloric acid to produce a compound represented by formula (III): 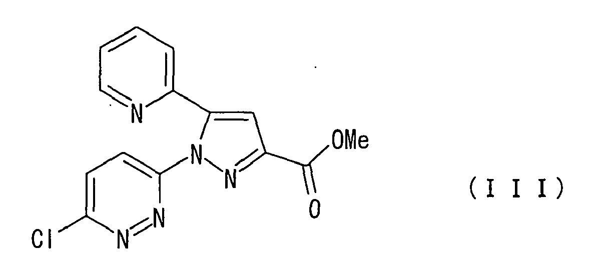 reacting this compound with sodium methoxide in methanol; treating the reaction mixture with sodium hydroxide to produce a compound represented by formula (II): 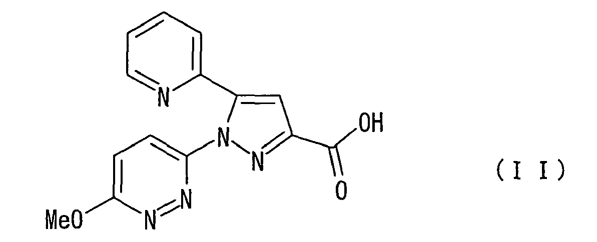 ; reacting this compound with N-methylpiperazine in acetonitrile in the presence of 1-hydroxybenzotriazol monohydrate and dicyclohexylcarbodiimide; and treating this reaction mixture with concentrated hydrochloric acid.

17. A method for producing a compound represented by formula (Ib): 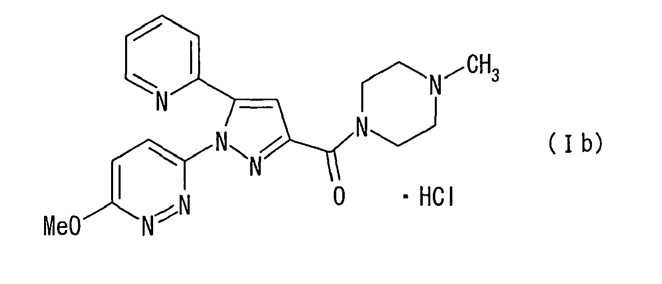 comprising the steps of reacting acetylpyridine with dimethyl oxalate in methanol in the presence of sodium methoxide to produce a compound represented by formula (IV): 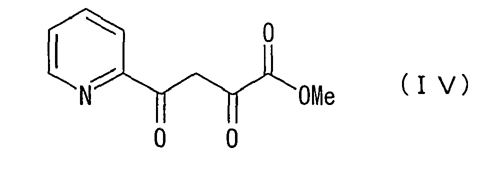 ; reacting this compound with 3-chloro-6-hydrazinopyridazine in methanol in the presence of concentrated hydrochloric acid to produce a compound represented by formula (III): 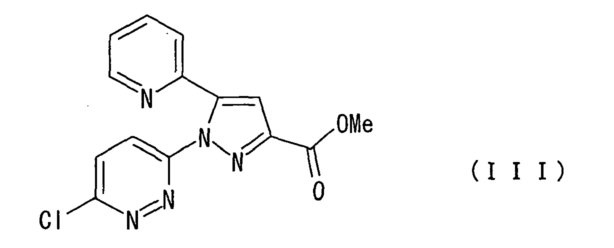 ; reacting this compound with sodium methoxide in methanol; treating the reaction mixture with sodium hydroxide to produce a compound represented by formula (II): 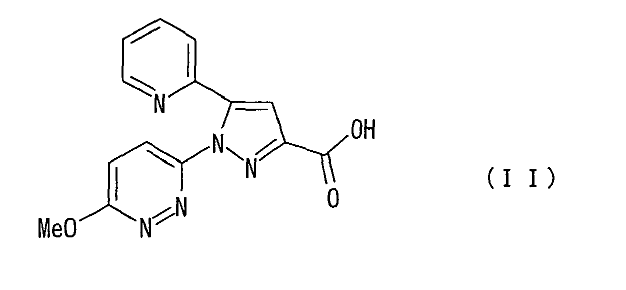 ; and reacting this compound with N-methylpiperazine in acetonitrile in the presence of 1-hydroxybenzotriazol monohydrate and dicyclohexylcarbodiimide; and treating this compound with concentrated hydrochloric acid.

18. A method for producing a compound represented by formula (Ib) : 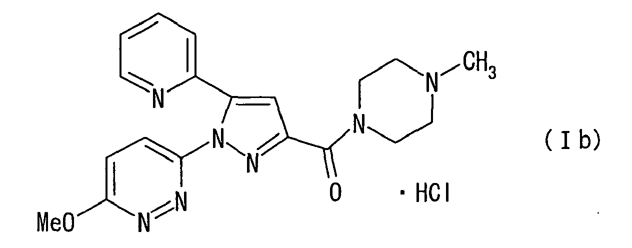 comprising the steps of reacting a compound represented by formula (IV) : 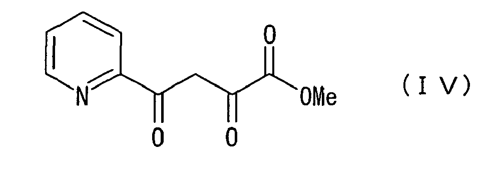 with 3-chloro-6-hydrazinopyridazine in methanol in the presence of methanesulfonic acid or in the presence of concentrated hydrochloric acid; adding sodium methoxide; treating the reaction mixture with sodium hydroxide to produce a compound represented by formula (II): 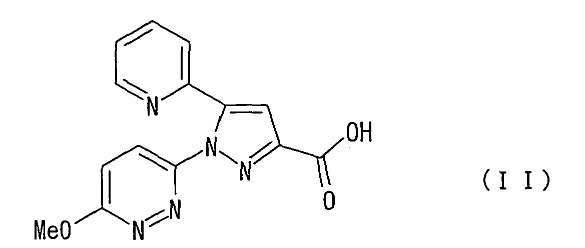 ; reacting this compound with N-methylpiperazine in acetonitrile and in the presence of 1-hydroxybenzotriazol monohydrate and dicyclohexylcarbodiimide; and treating the reaction mixture with concentrated hydrochloric acid.

19. A method for producing a compound represented by formula (Ib): 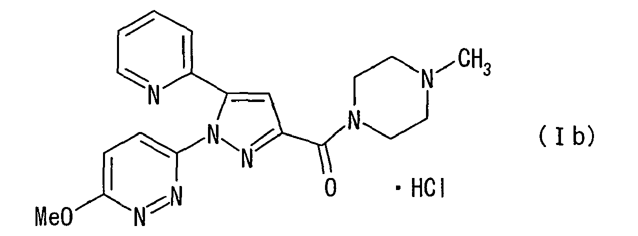 comprising the steps of reacting acetylpyridine with dimethyl oxalate in methanol in the presence of sodium methoxide to produce a compound represented by formula (IV): 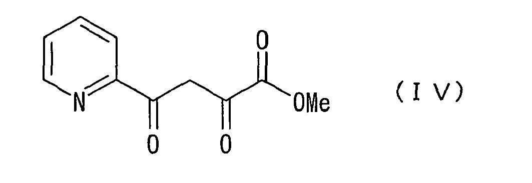 ; reacting this compound with 3-chloro-6-hydrazinopyridazine in methanol in the presence of methanesulfonic acid or in the presence of concentrated hydrochloric acid; adding sodium methoxide; treating the reaction mixture with sodium hydroxide to produce a compound represented by formula (II): 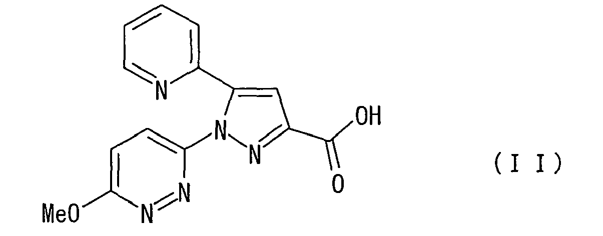 ; reacting this compound with N-methylpiperazine in acetonitrile in the presence of 1-hydroxybenzotriazol monohydrate and dicyclohexylcarbodiimide; and treating the reaction mixture with concentrated hydrochloric acid.

20. A method for producing a compound represented by formula (Ic) : 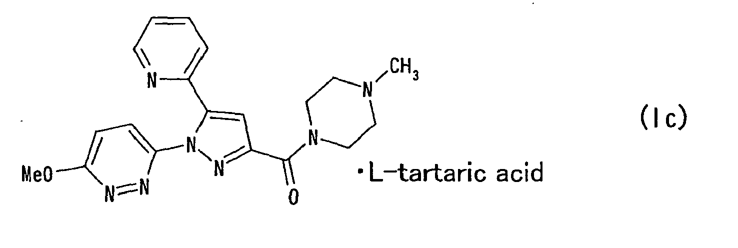 comprising the steps of reacting a compound represented by formula (II): 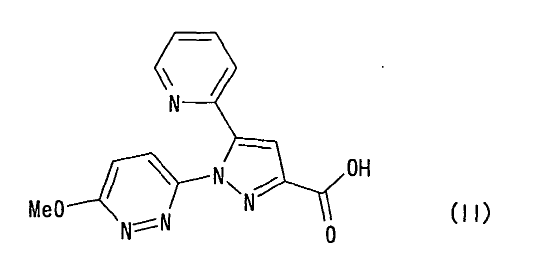 with N-methylpiperazine in acetonitrile in the presence of 1-hydroxybenzotriazol monohydrate and
1-ethyl-3-(3-dimethylaminopropyl)carbodiimide to produce a compound represented by formula (Ia): 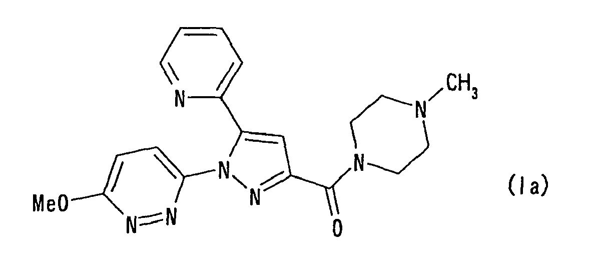 ; and reacting this compound with L-tartaric acid.

21. A method for producing a compound represented by formula (II): 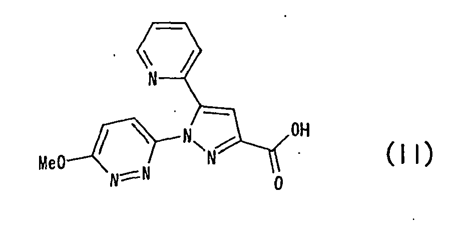 comprising the steps of reacting a compound represented by formula (III) : 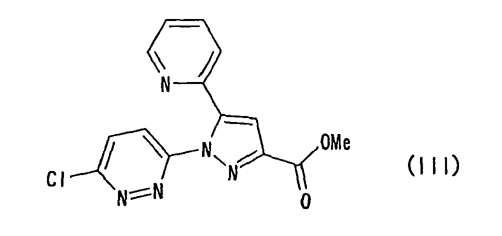 with sodium methoxide in methanol; and treating the reaction mixture with sodium hydroxide.

22. A method for producing a compound represented by formula (II) : 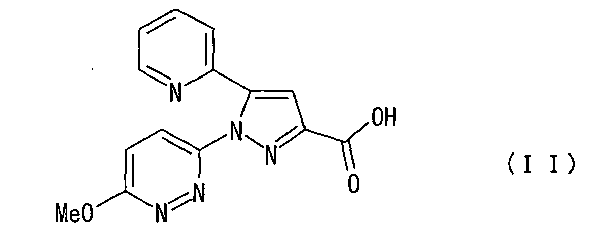 comprising the steps of reacting a compound represented by formula (IV) : 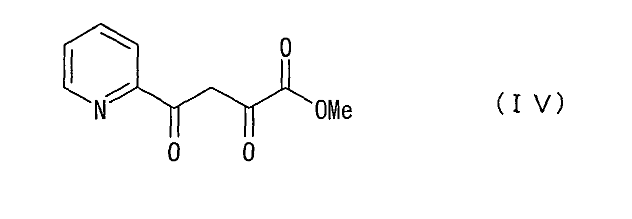 with 3-chloro-6-hydrazinopyridazine in methanol in the presence of methanesulfonic acid; adding sodium methoxide; and treating the reaction mixture with sodium hydroxide.

23. A method for producing a compound represented by formula (II) : 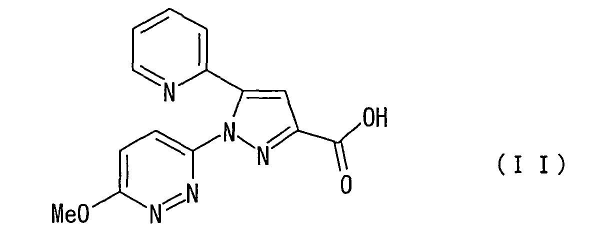 comprising the steps of reacting a compound represented by formula (IV) : 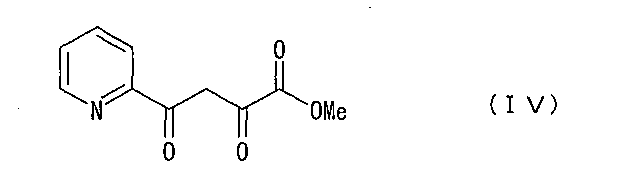 with 3-chloro-6-hydrazinopyridazine in methanol in the presence of concentrated hydrochloric acid; adding sodium methoxide; and treating the reaction mixture with sodium hydroxide.

24. A pharmaceutical composition comprising the compound of any one of claims 1 to 7 and a pharmaceutically acceptable carrier.

25. A medicament containing the pharmaceutical composition of claim 24.

26. A medicament containing the compound of any one of claims 1 to 7 as its effective component.

27. A medicament according to claim 25 or 26 wherein the medicament is used in preventing and/or treating an ischemic disease.

28. A method for preventing and/or treating an ischemic disease by administering an effective amount of the compound of any one of claims 1 to 7.

29. Use of the compound of any one of claims 1 to 7 in producing a medicament.
